# EUROPEAN PATENT SPECIFICATION

(11) **EP 4 010 323 B1**
(45) Date of publication and mention of the grant of the patent: **09.04.2025**
(21) Application number: 20753268.0
(22) Date of filing: 06.08.2020
(51) Int. Cl.: C07D 239/34, A01N 43/54

(54) **2-PHENOXY-PYRIMIDINE DERIVATIVES AS HERBICIDAL COMPOUNDS**
2-PHENOXY-PYRIMIDINDERIVATE ALS HERBIZIDE VERBINDUNGEN
DÉRIVÉS DE 2-PHÉNOXY-PYRIMIDINE EN TANT QUE COMPOSÉS HERBICIDES

(30) Priority: 09.08.2019 GB 201911429
(43) Date of publication of application: 15.06.2022
(73) Proprietor: Syngenta Crop Protection AG, 4058 Basel (CH)
(72) Inventor: WAILES, Jeffrey, Steven, Bracknell Berkshire RG42 6EY (GB); TATE, Joseph, Andrew, Bracknell Berkshire RG42 6EY (GB); INGRAM, Katharine, Mary, Bracknell Berkshire RG42 6EY (GB)
(74) Representative: SYNGENTA IP
(86) International application number: PCT/EP2020/072168
(87) International publication number: WO 2021/028316

(56) References cited:
- WO-A1-2015/089003
- WO-A1-2017/011288
- DE-A1- 10 256 354

## Description

The present invention relates to novel herbicidal compounds, to processes for their preparation, to herbicidal compositions which comprise the novel compounds, and to their use for controlling weeds, in particular in crops of useful plants, or for inhibiting plant growth.

WO2017/011288 discloses various aryloxypyrimidinyl ethers and their use as herbicides. The present invention relates to novel aryloxypyrimidinyl thioethers which show improved properties compared to the known ether compounds.

Thus, according to the present invention there is provided a compound of Formula (I): 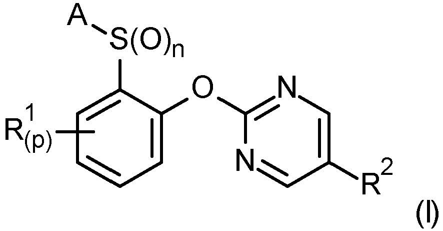 wherein
A is selected from the group consisting of C₃-C₈ alkyl, C₃-C₈ alkenyl, C₃-C₈ alkynyl, C₃-C₈ haloalkyl, C₃-C₈ haloalkenyl, C₃-C₈ haloalkynyl, C₁-C₄alkoxy-C₁-C₃alkyl-, C₁-C₄haloalkoxy-C₁-C₃alkyl-, C₁-C₄alkoxy-C₁-C₃haloalkyl- and - (CH₂)ₘR³;
R¹ is independently selected from the group consisting of halogen, -CN, nitro, C₁-C₄alkyl-, C₂-C₄alkenyl-, C₂-C₄alkynyl-, C₁-C₄haloalkyl-, C₁-C₄ alkoxy-, C₁-C₄haloalkoxy-, -S(O)ₙC₁-C₄alkyl and C₃-C₆cycloalkyl-;
R² is selected from the group consisting of halogen, -CN, nitro, C₁-C₄alkyl, C₂-C₄alkenyl-, C₂-C₄alkynyl-, C₁-C₄haloalkyl-, C₁-C₄alkoxy-, C₁-C₄haloalkoxy-, - S(O)ₙC₁-C₄alkyl and C₃-C₆cycloalkyl-;
m is 1, 2, 3 or 4;
n is 0, 1 or 2;
p is 0, 1 or 2;
R³ is selected from C₃-C₆ cycloalkyl, phenyl and a 5 or 6 membered heteroaryl which comprises from 1 to 4 heteroatoms each independently selected from the group consisting of oxygen, nitrogen and sulphur, and wherein said phenyl or heteroaryl groups are optionally substituted by one, two or three substituents independently selected from the group consisting of halogen, cyano, C₁-C₄ alkyl, C₁-C₄ haloalkyl, C₁-C₄ alkoxy and C₁-C₄ haloalkoxy;
or an agriculturally acceptable salt thereof.

Alkyl groups (e.g C₁-C₆alkyl) include, for example, methyl (Me, CH₃), ethyl (Et, C₂H₅), n-propyl (*n*-Pr), isopropyl (*i*-Pr), *n*-butyl (*n*-Bu), isobutyl (i-Bu), sec-butyl (s-Bu) and *tert*-butyl (*t*-Bu).

Alkenyl and alkynyl moieties can be in the form of straight or branched chains, and the alkenyl moieties, where appropriate, can be of either the (E)- or (Z)-configuration. Examples are vinyl, allyl and propargyl. Alkenyl and alkynyl moieties can contain one or more double and/or triple bonds in any combination.

Halogen (or halo) encompasses fluorine, chlorine, bromine or iodine. The same correspondingly applies to halogen in the context of other definitions, such as haloalkyl.

Haloalkyl groups (e.g C₁-C₆haloalkyl) are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, heptafluoro-n-propyl and perfluoro-n-hexyl.

Alkoxy groups (e.g C₁-C₄alkoxy-) are, for example, methoxy, ethoxy, propoxy, isopropoxy, n-butoxy, isobutoxy, sec-butoxy or tert-butoxy, preferably methoxy and ethoxy.

Alkoxyalkyl groups (e.g C₁-C₄alkoxy-C₁-C₃alkyl-) includes, for example, methoxymethyl, methoxyethyl, ethoxymethyl, ethoxyethyl, n-propoxymethyl, n-propoxyethyl, isopropoxymethyl or isopropoxyethyl.

Cycloalkyl groups (e.g C₃-C₆cycloalkyl-) include, for example cyclopropyl (c-propyl, c-Pr), cyclobutyl (c-butyl, c-Bu), cyclopentyl (c-pentyl) and cyclohexyl (c-hexyl) and may be substituted or unsubstituted as indicated.

C₁-C₆alkyl-S- (alkylthio) is, for example, methylthio, ethylthio, propylthio, isopropylthio, n-butylthio, isobutylthio, sec-butylthio or tert-butylthio, preferably methylthio or ethylthio.

C₁-C₆alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₆alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In one embodiment of the present invention there is provided a compound of Formula (Ia):

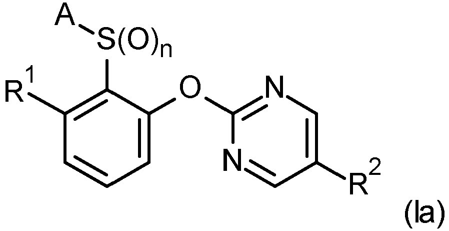

In one embodiment of the invention there is provided a compound of Formula (I) or Formula (la) wherein n is 0.

In one embodiment of the invention there is provided a compound of Formula (I) or Formula (la) wherein n is 1.

In one embodiment of the invention there is provided a compound of Formula (I) or Formula (la) wherein n is 2.

In another embodiment of the present invention there is provided a compound of Formula (I) or (la), wherein A is selected from the group consisting of C₃-C₈ alkyl, C₃-C₈ alkenyl, C₃-C₈ alkynyl, C₃-C₈ haloalkyl, C₃-C₈ haloalkenyl, C₃-C₈ haloalkynyl, C₁-C₄alkoxy-C₁-C₃alkyl-, C₁-C₄haloalkoxy-C₁-C₃alkyl- and C₁-C₄alkoxy-C₁-C₃haloalkyl-. In a more preferred embodiment of the present invention there is provided a compound of Formula (I) or Formula (la), wherein A is selected from the group consisting of C₃-C₈ alkyl, C₃-C₈ haloalkyl, C₃-C₈ haloalkenyl, C₃-C₈ haloalkynyl and C₁-C₄haloalkoxy-C₁-C₃alkyl-. In a further preferred embodiment A is selected from the group consisting of 3,3,3-trifluoroprop-1-yl, 1-butyl, 2-pentyl, 4,4,4-trifluorobut-1-yl, *E*-CH₂CH=CHCl, benzyl, -CH₂(4-Cl-C₆H₄), -CH₂(4-CN-C₆H₄), and CH₂(4-thiazolyl).

In one embodiment of the present invention there is provided compound of Formula (I) or Formula (la) wherein R¹ is halogen (e.g fluoro or chloro) or -CN.

In another embodiment, there is provided a compound of Formula (I) or Formula (la) wherein R² is selected from the group consisting of halogen, C₁-C₄ alkyl, C₂-C₄ alkenyl, C₂-C₄ alkynyl, C₁-C₄ haloalkyl, C₁-C₄ alkoxy and C₁-C₄ haloalkoxy. In a preferred embodiment R² is halogen, preferably chloro.

The compounds of Formula (I) according to the invention can be used as herbicides by themselves, but they are generally formulated into herbicidal compositions using formulation adjuvants, such as carriers, solvents and surface-active agents (SAA). Thus, the present invention further provides a herbicidal composition comprising a herbicidal compound according to the present invention and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

As outlined above, the compounds of the present invention may contain an asymmetric centre. As such, the compound of the present invention may be present in the composition as a racemic mixture of the two enantiomers. Alternatively, the compound of the present invention may be present in an enantiomer enriched form.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical and biological properties of the compound of Formula (I).

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from preformed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface-active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkyl naphthalenes) and other appropriate organic solvents which have a low solubility in water.

Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type.

Suitable SAAs of the cationic type include quaternary ammonium compounds (for example cetyltrimethyl ammonium bromide), imidazolines and amine salts.

Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols.

Suitable SAAs of the amphoteric type include betaines, propionates and glycinates.

Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols.

Suitable suspending agents include hydrophilic colloids (such as polysaccharides, polyvinylpyrrolidone or sodium carboxymethylcellulose) and swelling clays (such as bentonite or attapulgite).

The herbicidal compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, bensulfuron (including bensulfuron-methyl), bentazone, bicyclopyrone, bilanafos, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, diquat dibromide, diuron, ethalfluralin, ethofumesate, fenoxaprop (including fenoxaprop-P-ethyl), fenoxasulfone, fenquinotrione, fentrazamide, flazasulfuron, florasulam, florpyrauxifen (including florpyraxifen-benzyl), fluazifop (including fluazifop-P-butyl), flucarbazone (including flucarbazone-sodium), flufenacet, flumetsulam, flumioxazin, flupyrsulfuron (including flupyrsulfuron-methyl-sodium), fluroxypyr (including fluroxypyr-meptyl), fomesafen, foramsulfuron, glufosinate (including the ammonium salt thereof), glyphosate (including the diammonium, isopropylammonium and potassium salts thereof), halauxifen (including halauxifen-methyl), haloxyfop (including haloxyfop-methyl), hexazinone, hydantocidin, imazamox, imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimsulfuron, saflufenacil, sethoxydim, simazine, S-metalochlor, sulfentrazone, sulfosulfuron, tebuthiuron, tefuryltrione, tembotrione, terbuthylazine, terbutryn, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 3-[2-(3,4-dimethoxyphenyl)-6-methyl-3-oxo-pyridazine-4-carbonyl]bicyclo[3.2.1]octane-2,4-dione, 2-[2-(3,4-dimethoxyphenyl)-6-methyl-3-oxo-pyridazine-4-carbonyl]-5-methyl-cyclohexane-1,3-dione, 2-[2-(3,4-dimethoxyphenyl)-6-methyl-3-oxo-pyridazine-4-carbonyl]cyclohexane-1,3-dione, 2-[2-(3,4-dimethoxyphenyl)-6-methyl-3-oxo-pyridazine-4-carbonyl]-5,5-dimethyl-cyclohexane-1,3-dione, 6-[2-(3,4-dimethoxyphenyl)-6-methyl-3-oxo-pyridazine-4-carbonyl]-2,2,4,4-tetramethyl-cyclohexane-1,3,5-trione, 2-[2-(3,4-dimethoxyphenyl)-6-methyl-3-oxo-pyridazine-4-carbonyl]-5-ethyl-cyclohexane-1,3-dione, 2-[2-(3,4-dimethoxyphenyl)-6-methyl-3-oxo-pyridazine-4-carbonyl]-4,4,6,6-tetramethyl-cyclohexane-1,3-dione, 2-[6-cyclopropyl-2-(3,4-dimethoxyphenyl)-3-oxo-pyridazine-4-carbonyl]-5-methyl-cyclohexane-1,3-dione, 3-[6-cyclopropyl-2-(3,4-dimethoxyphenyl)-3-oxo-pyridazine-4-carbonyl]bicyclo[3.2.1]octane-2,4-dione, 2-[6-cyclopropyl-2-(3,4-dimethoxyphenyl)-3-oxo-pyridazine-4-carbonyl]-5,5-dimethyl-cyclohexane-1,3-dione, 6-[6-cyclopropyl-2-(3,4-dimethoxyphenyl)-3-oxo-pyridazine-4-carbonyl]-2,2,4,4-tetramethyl-cyclohexane-1,3,5-trione, 2-[6-cyclopropyl-2-(3,4-dimethoxyphenyl)-3-oxo-pyridazine-4-carbonyl]cyclohexane-1,3-dione, 4-[2-(3,4-dimethoxyphenyl)-6-methyl-3-oxo-pyridazine-4-carbonyl]-2,2,6,6-tetramethyl-tetrahydropyran-3,5-dione and 4-[6-cyclopropyl-2-(3,4-dimethoxyphenyl)-3-oxo-pyridazine-4-carbonyl]-2,2,6,6-tetramethyl-tetrahydropyran-3,5-dione.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or N-(2-methoxybenzoyl)-4-[(methyl-aminocarbonyl)amino]benzenesulfonamide.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The present invention still further provides a method of controlling weeds at a locus, said method comprising application to the locus of a weed controlling amount of a composition comprising a compound of Formula (I). Moreover, the present invention may further provide a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. It is noted that the compounds of the present invention show a much-improved selectivity compared to know, structurally similar compounds. Generally the plants to be controlled are unwanted plants (weeds). 'Locus' means the area in which the plants are growing or will grow. The application may be applied to the locus pre-emergence and/or postemergence of the crop plant. Some crop plants may be inherently tolerant to herbicidal effects of compounds of Formula (I). Preferred crop plants include maize, wheat, barley and rice.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to other herbicides or classes of herbicides (e.g. ALS-, GS-, EPSPS-, PPO-, HPPD-, -PDS and ACCase-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compositions can be used to control unwanted plants (collectively, 'weeds'). The weeds to be controlled may be both monocotyledonous species, for example *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

In a further aspect of the present invention there is provided the use of a compound of Formula (I) as defined herein as a herbicide.

The compounds of the present invention can be prepared according to the following schemes.

Typical abbreviations used throughout are as follows:
Ac = acetate
br = broad
d = doublet
DABCO = 1,4-diazabicyclo[2.2.2]octane
DCM = dichloromethane
DEAD = diethylazodicarboxylate
DIAD = diisopropylazodicarboxylate
DMF = *N*,*N*-dimethylformamide
Et = ethyl
Et₂O = diethyl ether
EtOAc = ethyl acetate
HPLC = high performance liquid chromatography
LiHMDS = lithium bis(trimethylsilyl)amide
m = multiplet
Me = methyl
MeCN = acetonitrile
MeOH = methanol
Ms = mesylate
PG = protecting group
Ph = phenyl
Ph₂O = diphenyl ether
q = quartet
RT = room temperature
s = singlet
t = triplet
TBAF = tetrabutylammonium fluoride
TIPS = triisopropylsilyl
Tf = triflate
THF = tetrahydrofuran
Ts = tosylate

### Processes for preparation of compounds, e.g. compounds of formula (I)

Processes for preparation of compounds, e.g. compounds of formula (I) (which optionally can be an agrochemically acceptable salt thereof), are now described, and form further aspects of the present invention.

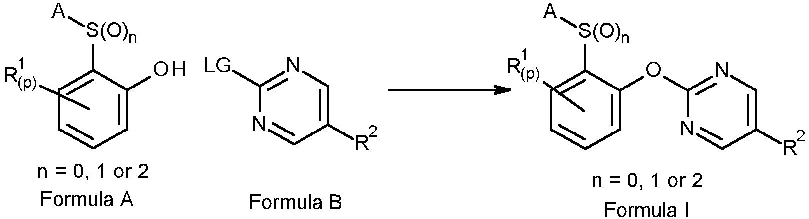

A compound of Formula I may be prepared from a compound of Formula A by reaction with a compound of Formula B (where LG represents a suitable leaving group, such as Br, Cl, F or SO₂Me) optionally in the presence of a suitable base and in a suitable solvent. Suitable bases may include K₂CO₃ or Cs₂CO₃. Suitable solvents may include DMF or MeCN. Compounds of Formula B are commercially available or may be prepared by methods known in the literature.

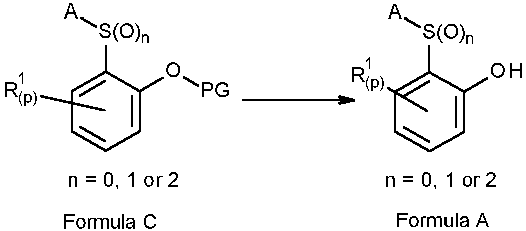

A compound of Formula A may be prepared from a compound of Formula C (where PG represents a suitable protecting group, such as Me or Ac) by a deprotection reaction. Suitable conditions for deprotection when PG = Me include the use of BBr₃ in a suitable solvent, such as DCM. Suitable conditions for deprotection when PG = Ac include the use of K₂CO₃ or NH₄OAc in a suitable solvent, such as MeOH or MeOH/H₂O.

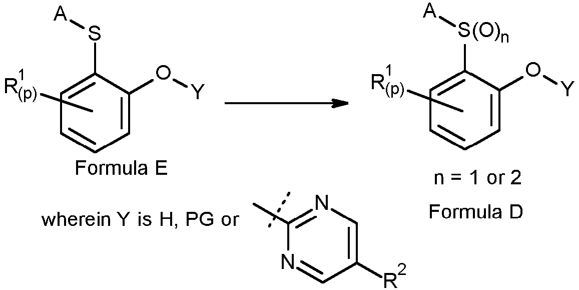

A compound of Formula D (which includes compounds of Formula I, A and C where n = 1 or 2) may be prepared from a compound of Formula E (which includes compounds of Formulas I, A and C where n = 0) by reaction with a suitable oxidizing reagent in a suitable solvent. Suitable oxidizing reagents may include meta-chloroperoxybenzoic acid. Suitable solvents may include DCM. Suitable conditions for preparing compounds of Formula D where n = 1 may include the use of one molar equivalent of oxidizing reagent. Suitable conditions for preparing compounds of Formula D where n = 2 may include the use of two or more molar equivalents of oxidizing reagent. 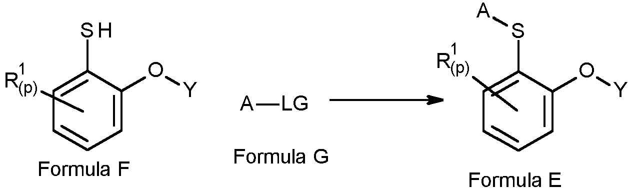 wherein Y is H, PG or

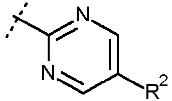

A compound of Formula E (which includes compounds of Formulas I, A and C where n = 0) may be prepared from a compound of Formula F by reaction with an alkylating reagent of Formula G (where LG represents a suitable leaving group, such as Br, Cl, OMs or OTs) optionally in the presence of a suitable base and in a suitable solvent. Suitable bases may include K₂CO₃ or Cs₂CO₃. Suitable solvents may include DMF or MeCN. Compounds of Formula G are commercially available or may be prepared by methods known in the literature. Compounds of Formula F where Y = H or PG (e.g. Me) are commercially available or may be prepared by methods known in the literature (see below for examples). 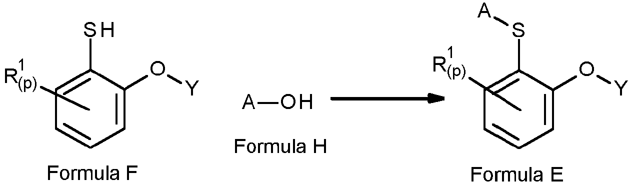 wherein Y is H, PG or

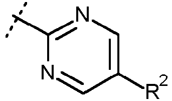

In an alternative approach, a compound of Formula E (which includes compounds of Formulas I, A and C where n = 0) may be prepared from a compound of Formula F by Mitsunobu reaction with an alcohol of Formula H in the presence of an azodicarboxylate reagent and a phosphine in a suitable solvent. Suitable azodicarboxylate reagents may include diethylazodicarboxylate (DEAD) or diisopropylazodicarboxylate (DIAD). Suitable phosphines may include triphenylphosphine. Suitable solvents may include THF. Compounds of Formula F where Y = H or PG (e.g. Me) are commercially available or may be prepared by methods known in the literature (see below for examples).

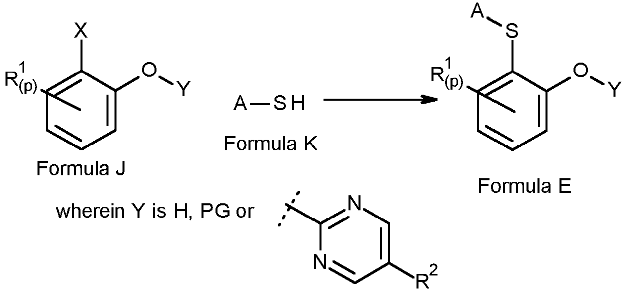

In a further alternative approach, a compound of Formula E (which includes compounds of Formulas I, A and C where n = 0) may be prepared from a compound of Formula J (where X represents a suitable halogen such as F, Cl, Br or I or a suitable pseudohalogen such as OTf) by reaction with a thiol of Formula K optionally in the presence of a suitable catalyst/ligand combination, in the presence of a suitable base and in a suitable solvent. Suitable catalysts may include [(4,5-bis(diphenylphosphino)-9,9-dimethylxanthene)-2-(2'-amino-1,1'-biphenyl)]palladium(II) methanesulfonate. Suitable bases may include N,N-diisopropylethylamine or K₂CO₃. Suitable solvents may include 1,4-dioxane or DMF. Compounds of Formula K are commercially available or may be prepared by methods known in the literature. Compounds of Formula J where Y = H or PG (e.g. Me) are commercially available or may be prepared by methods known in the literature. 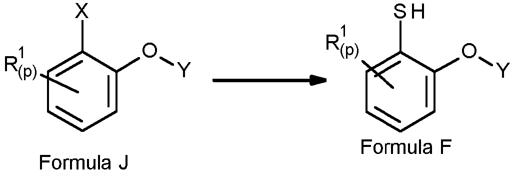 wherein Y is H, PG or

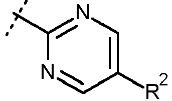

A compound of Formula F may be prepared from a compound of Formula J (where X represents a suitable halogen such as F or Cl) by a nucleophilic aromatic substitution reaction with H₂S or a suitable H₂S surrogate optionally in the presence of a suitable base and in a suitable solvent. Suitable H₂S surrogates may include Na₂S, NaSH or Li₂S. Suitable solvents may include DMF. Compounds of Formula J where Y = H or PG (e.g. Me) are commercially available or may be prepared by methods known in the literature. 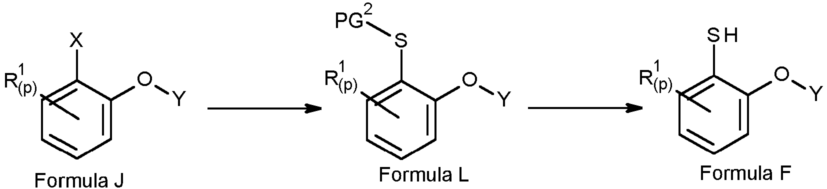 wherein Y is H, PG or

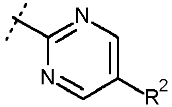

In an alternative approach, a compound of Formula F may be prepared from a compound of Formula J (where X represents a suitable halogen such as F, CI, Br or I or a suitable pseudohalogen such as OTf) in a two-step sequence. In the first step, a compound of Formula L (where PG² represents a suitable protecting group, such as TIPS) may be prepared from a compound of Formula J by reaction with an H₂S surrogate optionally in the presence of a suitable catalyst/ligand combination, in the presence of a suitable base and in a suitable solvent. Suitable H₂S surrogates may include TIPS-SH. Suitable catalysts may include a catalyst consisting of Pd(OAc)₂ and (2R)-1-[(1R)-1-[bis(1,1-dimethylethyl)phosphino]ethyl]-2-(dicyclohexylphosphino)ferrocene. Suitable bases may include LiHMDS. Suitable solvents may include toluene. In the second step, a compound of Formula F may be prepared from a compound of Formula L by a deprotection reaction. Suitable conditions for deprotection when PG² = TIPS may include the use of TBAF in a suitable solvent, such as toluene. Compounds of Formula J where Y = H or PG (e.g. Me) are commercially available or may be prepared by methods known in the literature. 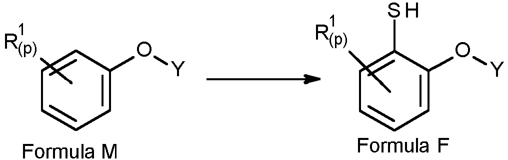 wherein Y is H, PG or

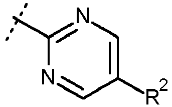

In a further alternative approach, a compound of Formula F may be prepared from a compound of Formula M by ortho-lithiation with a suitable alkyllithium reagent and subsequent reaction with elemental sulfur in a suitable solvent at a suitable reaction temperature. Suitable alkyllithium reagents may include n-butyllithium. Suitable solvents may include THF. Suitable reaction temperatures may be between -78 °C and 20 °C. Compounds of Formula M where Y = H or PG (e.g. Me) are commercially available or may be prepared by methods known in the literature.

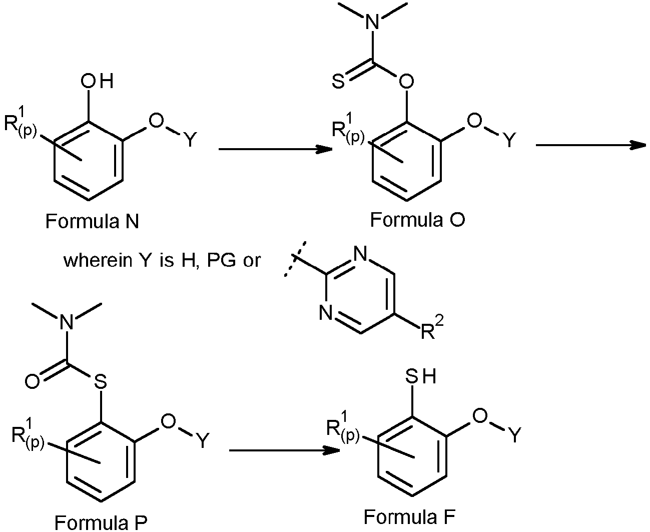

In a yet further alternative approach, a compound of Formula F may be prepared in a three-step sequence from a compound of Formula N. In the first step, a compound of Formula O may be prepared from a compound of Formula N by reaction with N,N-dimethyl thiocarbamoyl chloride in the presence of a suitable base and in a suitable solvent. Suitable bases may include DABCO or NaH. Suitable solvents may include DMF. In the second step, a compound of Formula P may be prepared by Newman-Kwart rearrangement of a compound of Formula O under a range of suitable conditions. Suitable conditions for rearrangement may include heating at temperatures ranging from 180 °C to 300 °C either neat, or in a suitable solvent, such as Ph₂O. In the third step, a compound of Formula F may be prepared from a compound of Formula P by reaction with NaOH or KOH in a suitable solvent. Suitable solvents may include H₂O or MeOH. Compounds of Formula N where Y = H or PG (e.g. Me) are commercially available or may be prepared by methods known in the literature.

The following non-limiting examples provide specific synthesis methods for representative compounds of the present invention, as referred to in the Tables below.

### Example 1: Synthesis of 3-(5-chloropyrimidin-2-yl)oxy-2-(4,4,4-trifluorobutylsulfanyl)benzonitrile - Compound A28.

### Step 1: Synthesis of 3-methoxy-2-(4,4,4-trifluorobutylsulfanyl)benzonitrile

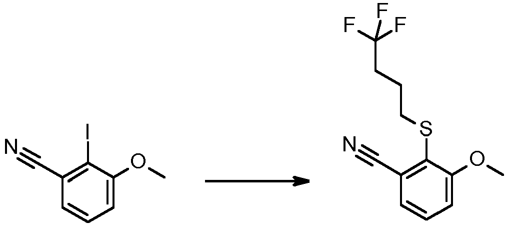

To a stirred solution of 2-iodo-3-methoxy-benzonitrile (1.0 g, 3.9 mmol) and N,N-diisopropylethylamine (1.0 g, 1.3 mL, 7.7 mmol) in 1,4-dioxane (15 mL) under nitrogen was added [(4,5-bis(diphenylphosphino)-9,9-dimethylxanthene)-2-(2'-amino-1,1'-biphenyl)]palladium(II) methanesulfonate (0.39 g, 0.39 mmol) and 4,4,4-trifluorobutane-1-thiol (0.61 g, 4.2 mmol). The reaction was heated at 120°C for 2 hours by microwave irradiation, after which the solvent was removed under reduced pressure. The crude residue was purified by flash chromatography on silica gel using a gradient of 0-30% ethyl acetate in cyclohexane as eluent to give the desired product (0.95 g, 72 %).

¹H NMR (400 MHz, CDCl₃) δ 7.44 - 7.37 (m, 1H), 7.30 (dd, 1H), 7.12 (dd, 1H), 3.94 (s, 3H), 3.01 (t, 2H), 2.40 - 2.24 (m, 2H), 1.83 - 1.70 (m, 2H)

### Step 2: Synthesis of 3-hydroxy-2-(4,4,4-trifluorobutylsulfanyl)benzonitrile

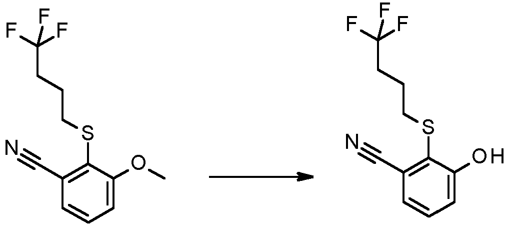

To a stirred solution of 3-methoxy-2-(4,4,4-trifluorobutylsulfanyl)benzonitrile (0.95 g, 3.5 mmol) in DCM (35 mL) at 0°C under an atmosphere of N₂ was added BBr₃ (8.6 mL of a 1M solution in DCM, 8.6 mmol). The reaction was allowed to warm to room temperature over 1 hour and was then stirred overnight after which the mixture was quenched with saturated NaHCO₃ solution and extracted with DCM (x3). The combined organic extracts were passed through a hydrophobic frit and concentrated under reduced pressure. The crude residue was purified by flash chromatography on silica gel using a gradient of 0-60% EtOAc in cyclohexane as eluent to give the desired product (0.76 g, 84 %).

¹H NMR (400 MHz, CDCl₃) δ 7.42 - 7.37 (m, 1H), 7.32 (dd, 1H), 7.26 (dd, 1H), 6.93 (br s, 1H), 2.92 (t, 2H), 2.34 - 2.20 (m, 2H), 1.92 - 1.81 (m, 2H)

### Step 3: Synthesis of 3-(5-chloropyrimidin-2-yl)oxy-2-(4,4,4-trifluorobutylsulfanyl)benzonitrile.

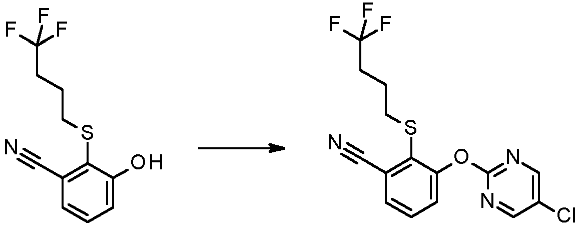

To a stirred solution of 3-hydroxy-2-(4,4,4-trifluorobutylsulfanyl)benzonitrile (0.30 g, 1.1 mmol) and potassium carbonate (0.32 g, 2.3 mmol) in dimethylformamide (3 mL) was added 2,5-dichloropyrimidine (0.21 g, 1.4 mmol). The reaction was heated at 80°C for 4 hours and then allowed to cool to RT. The reaction mixture was diluted with EtOAc and H₂O, the phases were separated and the aqueous phase was extracted with further EtOAc. The combined organics were dried over MgSO₄, filtered and evaporated to dryness under reduced pressure. The crude residue was purified by flash chromatography over silica gel using a gradient of 0-30% EtOAc in cyclohexane as eluent to give the desired product (0.33 g, 77 %).

¹H NMR (400 MHz, CDCl₃) δ 8.48 (s, 2H), 7.66 (dd, 1H), 7.53 (t, 1H), 7.48 - 7.42 (m, 1H), 3.05 - 2.99 (m, 2H), 2.35 - 2.17 (m, 2H), 1.81 - 1.72 (m, 2H)

### Example 2: Synthesis of 3-(5-chloropyrimidin-2-yl)oxy-2-(4,4,4-trifluorobutylsulfinyl)benzonitrile - Compound A29.

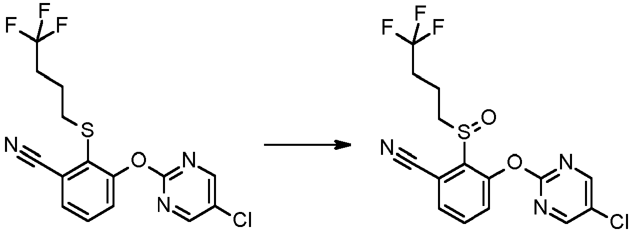

To a stirred solution of 3-(5-chloropyrimidin-2-yl)oxy-2-(4,4,4-trifluorobutylsulfanyl)benzonitrile (0.10 g, 0.27 mmol) in DCM (2.7 mL) at 0°C was added 3-chloroperbenzoic acid (≤77 %, 0.060 g, 0.27 mmol). The reaction was stirred at 0°C for 20 minutes and then diluted with DCM and saturated Na₂S₂O₅ solution. The phases were separated and the organic phase washed with further portions of saturated Na₂S₂O₅ solution (x2). The organic phase was then washed with saturated NaHCO₃ solution, passed through a hydrophobic frit and concentrated under reduced pressure. The crude residue was purified by flash chromatography on silica gel using a gradient of 0-100% EtOAc in cyclohexane as eluent to give the desired product (0.089 g, 85 %).

¹H NMR (400 MHz, CDCl₃) δ 8.50 (s, 2H), 7.80 (dd, 1H), 7.70 (t, 1H), 7.47 (dd, 1H), 3.50 - 3.41 (m, 1H), 3.23 - 3.12 (m, 1H), 2.40 - 2.25 (m, 2H), 2.19 - 2.06 (m, 2H)

### Example 3: Synthesis of 3-(5-chloropyrimidin-2-yl)oxy-2-(4,4,4-trifluorobutylsulfonyl)benzonitrile - Compound A30.

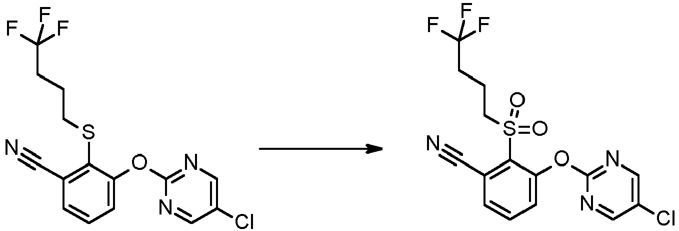

To a stirred solution of 3-(5-chloropyrimidin-2-yl)oxy-2-(4,4,4-trifluorobutylsulfanyl)benzonitrile (0.13 g, 0.35 mmol) in DCM (3.5 mL) at 0°C was added 3-chloroperbenzoic acid (≤77 %, 0.390 g, 1.74 mmol). The reaction was stirred at 0°C for 3 hours and then diluted with DCM and saturated Na₂S₂O₅ solution. The phases were separated and the organic phase washed with further portions of saturated Na₂S₂O₅ solution (x2). The organic phase was then washed with saturated NaHCO₃ solution, passed through a hydrophobic frit and concentrated under reduced pressure. The crude residue was purified by flash chromatography on silica gel using a gradient of 0-100% EtOAc in cyclohexane as eluent to give the desired product (0.129 g, 91 %).

¹H NMR (400 MHz, CDCl₃) δ 8.49 (s, 2H), 7.90 - 7.83 (m, 2H), 7.61 (dd, 1H), 3.59 (t, 2H), 2.42 - 2.29 (m, 2H), 2.16 - 2.07 (m, 2H)

### Example 4: Synthesis of 5-chloro-2-[3-chloro-2-(3,3,3-trifluoropropylsulfanyl) phenoxy]pyrimidine - Compound A10.

### Step 1: Synthesis of 1-chloro-3-methoxy-2-(3,3,3-trifluoropropylsulfanyl) benzene

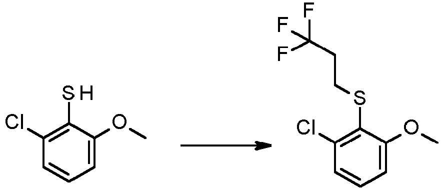

To a stirred solution of 2-chloro-6-methoxy-benzenethiol (0.30 g, 1.7 mmol) and potassium carbonate (0.47 g, 3.4 mmol) in dimethylformamide (3 mL) was added 1,1,1-trifluoro-3-iodo-propane (0.46 g, 0.24 mL, 2.1 mmol). The reaction was stirred overnight at room temperature and then diluted with Et₂O and water. The phases were separated and the aqueous was re-extracted with Et₂O. The combined organics were dried over MgSO₄ and evaporated to dryness under reduced pressure. The crude residue was purified by flash chromatography over silica gel using a gradient of 0-20% EtOAc in cyclohexane as eluent to give the desired product (0.44 g, 94 %).

¹H NMR (400 MHz, CDCl₃) δ 7.32 - 7.28 (m, 1H), 7.14 (dd, 1H), 6.87 (dd, 1H), 3.95 (s, 3H), 3.09 - 3.02 (m, 2H), 2.41 - 2.27 (m, 2H)

### Step 2: Synthesis of 3-chloro-2-(3,3,3-trifluoropropylsulfanyl)phenol

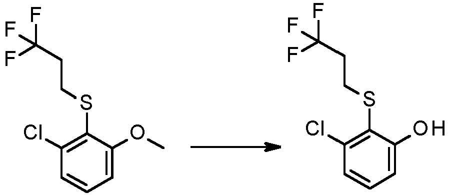

To a stirred solution of 1-chloro-3-methoxy-2-(3,3,3-trifluoropropylsulfanyl)benzene (0.40 g, 1.5 mmol) in DCM (15 mL) at 0°C under an atmosphere of N₂ was added BBr₃ (3.7 mL of a 1M solution in DCM, 3.7 mmol). The reaction was allowed to warm to room temperature and was stirred for 4 hours after which the mixture was quenched with saturated NaHCO₃ solution and extracted with DCM (x2). The combined organic extracts were passed through a hydrophobic frit and concentrated under reduced pressure. The crude residue was purified by flash chromatography on silica gel using a gradient of 0-15% EtOAc in cyclohexane as eluent to give the desired product (0.30 g, 78 %).

¹H NMR (400 MHz, CDCl₃) δ 7.25 (t, 1H), 7.06 (dd, 1H), 6.96 (dd, 1H), 6.94 (s, 1H), 3.00 - 2.92 (m, 2H), 2.42 - 2.28 (m, 2H)

### Step 3: Synthesis of 5-chloro-2-[3-chloro-2-(3,3,3-trifluoropropylsulfanyl) phenoxy]pyrimidine.

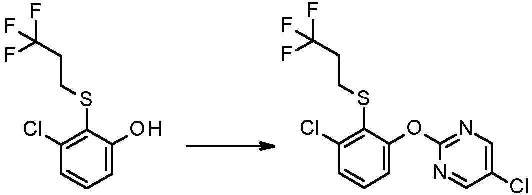

To a stirred solution of 3-chloro-2-(3,3,3-trifluoropropylsulfanyl)phenol (0.18 g, 0.70 mmol) and potassium carbonate (0.19 g, 1.4 mmol) in dimethylformamide (1.8 mL) was added 2,5-dichloropyrimidine (0.13 g, 0.84 mmol). The reaction was heated at 80°C for 3 hours and then allowed to cool to RT. The reaction mixture was diluted with EtOAc and H₂O, the phases were separated and the aqueous phase was extracted with further EtOAc. The combined organics were dried over MgSO₄, filtered and evaporated to dryness under reduced pressure. The crude residue was purified by flash chromatography over silica gel using a gradient of 0-20% EtOAc in cyclohexane as eluent to give the desired product (0.16 g, 63 %).

¹H NMR (400 MHz, CDCl₃) δ 8.48 (s, 2H), 7.49 - 7.43 (m, 1H), 7.43 - 7.37 (m, 1H), 7.18 (dd, 1H), 3.01 - 2.95 (m, 2H), 2.36 - 2.23 (m, 2H)

### Example 5: Synthesis of 5-chloro-2-[2-(4,4,4-trifluorobutylsulfanyl)phenoxy] pyrimidine - Compound A4.

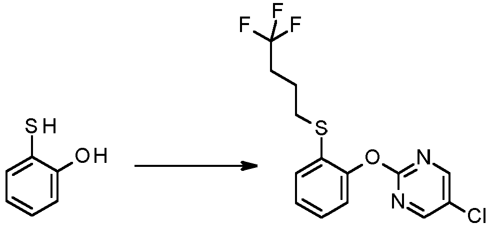

To a stirred solution of 2-sulfanylphenol (0.75 g, 0.60 mL, 5.9 mmol) and potassium carbonate (0.82 g, 5.9 mmol) in dimethylformamide (7.5 mL) was added 4-bromo-1,1,1-trifluoro-butane (1.14 g, 0.74 mL, 5.9 mmol). The reaction was stirred at room temperature for 1.5 hours after which potassium carbonate (0.90 g, 6.5 mmol) and 2,5-dichloropyrimidine (1.06 g, 7.1 mmol) were added. The reaction was heated at 80°C for 1 hour and then allowed to cool to RT. The reaction mixture was diluted with Et₂O and H₂O, the phases were separated and the aqueous phase was extracted with further Et₂O. The combined organics were dried over MgSO₄, filtered and evaporated to dryness under reduced pressure. The crude residue was purified by flash chromatography over silica gel using a gradient of 0-20% EtOAc in cyclohexane as eluent to give the desired product (1.38 g, 66 %).

¹H NMR (400 MHz, CDCl₃) δ 8.48 (s, 2H), 7.48 (dd, 1H), 7.40 - 7.31 (m, 1H), 7.31 - 7.24 (m, 1H), 7.19 (dd, 1H), 2.92 (t, 2H), 2.25 - 2.12 (m, 2H), 1.85 - 1.77 (m, 2H)

### Example 6: Synthesis of 5-chloro-2-[3-chloro-2-(4,4-difluorobutylsulfanyl) phenoxy]pyrimidine - Compound A105.

### Step 1: Synthesis of 2-[3-(2-chloro-6-methoxy-phenyl)sulfanylpropyl]-1,3-dioxolane

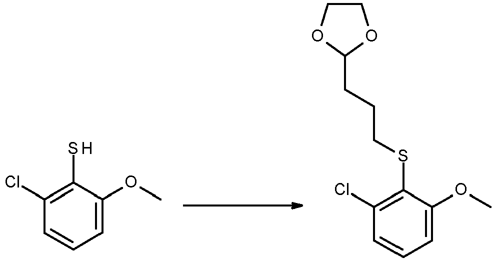

To a stirred solution of 2-chloro-6-methoxybenzenethiol (0.75g, 4.29 mmol) and 2-(3-chloropropyl)-1,3-dioxolane (0.776g, 5.15 mmol) in DMF (7.5 mL) was added K₂CO₃ (1.19g, 8.59 mmol). The reaction was stirred overnight at RT, then diluted with water and extracted with EtOAc (x3). The combined organic extracts were washed with brine, dried over MgSO₄ and evaporated to dryness under reduced pressure. The crude product was purified by flash chromatography on silica gel using a gradient of 0-20% EtOAc/cyclohexane as eluent to give the desired product (0.72g, 58%) as a colourless oil.

¹H NMR (400 MHz, CDCl₃) d = 7.19 (t, 1H), 7.07 (d, 1H), 6.79 (d, 1H), 4.81 (t, 1H), 3.94-3.85 (m, 2H), 3.88 (s, 3H), 3.84-3.78 (m, 2H), 2.90 (t, 2H), 1.82-1.73 (m, 2H), 1.68-1.58 (m, 2H)

### Step 2: Synthesis of 4-(2-chloro-6-methoxy-phenyl)sulfanylbutanal

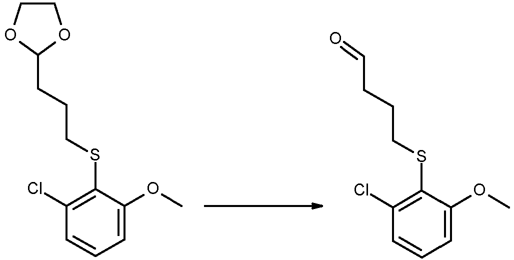

To a solution of 2-[3-(2-chloro-6-methoxy-phenyl)sulfanylpropyl]-1,3-dioxolane (0.72g, 2.49 mmol) in CH₃CN (6 mL) was added 2M HCl (2 mL). The reaction was stirred at RT overnight, further 2M HCl was added (1 mL) and the reaction heated at 60°C overnight. The reaction was allowed to cool to RT, diluted with water and extracted with EtOAc (x3). The combined organic extracts were washed with brine, dried over MgSO₄ and evaporated to dryness under reduced pressure to give the crude product (0.64g) as an orange oil which was used without further purification.

¹H NMR (400 MHz, CDCl₃) δ = 9.75 (s, 1H), 7.19 (t, 1H), 7.07 (d, 1H), 6.80 (d, 1H), 3.89 (s, 3H), 2.90 (t, 2H), 2.63 (t, 2H), 1.82-1.72 (m, 2H)

### Step 3: Synthesis of 1-chloro-2-(4,4-difluorobutylsulfanyl)-3-methoxy-benzene

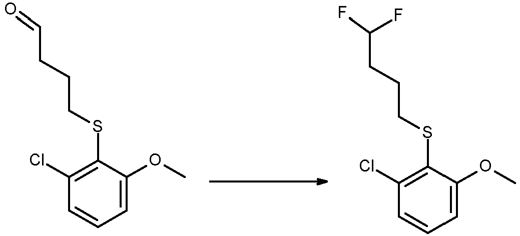

To a stirred solution of 4-(2-chloro-6-methoxy-phenyl)sulfanylbutanal (644mg, 2.63 mmol) in DCM (10 mL) was added dropwise diaminosulfur trifluoride (0.773 mL, 5.26 mmol). The reaction was stirred at RT for 2 hours, then cooled to 0°C, quenched with water, diluted with saturated aqueous sodium bicarbonate solution then extracted with EtOAc (x3). The combined organic extracts were washed with brine, dried over MgSO₄ and evaporated to dryness under reduced pressure. The crude product was purified by flash chromatography on silica gel using a gradient of 0-50% EtOAc/cyclohexane as eluent to give the desired product (0.395g, 56%) as a pale-yellow oil.

¹H NMR (400 MHz, CDCl₃) d = 7.19 (t, 1H), 7.09 (d, 1H), 6.80 (d, 1H), 5.78 (tt, 1H), 3.90 (s, 3H), 2.90 (t, 3H), 1.91-2.09 (m, 2H), 1.69-1.58 (m, 2H)

### Step 4: Synthesis of 3-chloro-2-(4,4-difluorobutylsulfanyl)phenol

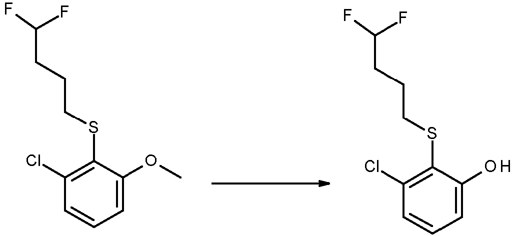

To a stirred solution of 1-chloro-2-(4,4-difluorobutylsulfanyl)-3-methoxybenzene (100mg, 0.396 mmol) in DMF (1.14 mL) at RT, under an atmosphere of N₂ was added 1-dodecanethiol (0.193 mL, 0.791 mmol) and LiO*^{t}*Bu (0.79 ml of a 1M solution in THF, 0.79 mmol). The reaction was heated at 100°C for 90 minutes, allowed to cool to RT, diluted with water and extracted with EtOAc (x3). The combined organic extracts were washed with brine, dried over MgSO₄ and evaporated to dryness under reduced pressure. The crude product was purified by flash chromatography on silica gel using a gradient of 0-30% EtOAc/cyclohexane as eluent to give the desired product as a mixture with 1-dodecanethiol which was used without further purification.

¹H NMR (400 MHz, CDCl₃) δ 7.19 (t, 1H), 7.02 (d, 1H), 7.00 (s, 1H), 6.91 (d, 1H), 5.79 (tt, 1H), 2.81 (t, 2H), 2.03-1.88 (m, 2H), 1.79-1.67 (m, 2H)

### Step 5: Synthesis of 5-chloro-2-[3-chloro-2-(4,4-difluorobutylsulfanyl)phenoxy] pyrimidine - Compound A105.

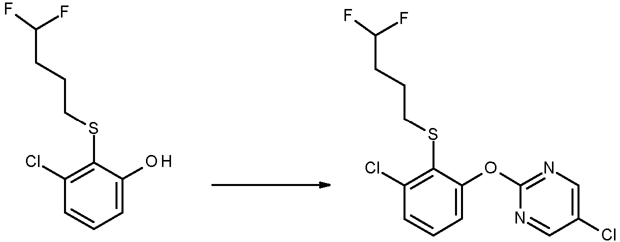

A mixture of 3-chloro-2-(4,4-difluorobutylsulfanyl)phenol (0.469g, 1.86 mmol), 2,5-dichloropyrimidine (0.332g, 2.23 mmol), K₂CO₃ (0.513g, 3.71 mmol) in DMF (3 mL) was heated at 140°C under microwave irradiation for 30 minutes. The reaction was diluted with water and extracted with EtOAc (x3). He combined organic extracts were washed with brine, dried over MgSO₄ and evaporated to dryness under reduced pressure. The crude product was purified by flash chromatography on silica gel using a gradient of 0-30% EtOAc/cyclohexane as eluent to give the desired product (.0487g, 72%) as an off-white semi-solid.

¹H NMR (400 MHz, CDCl₃) δ 8.47 (s, 2H), 7.41 (d, 1H), 7.33 (t, 1H), 7.13 (d, 1H), 5.78 (tt, 1H), 2.89 (t, 2H), 2.00-1.85 (m, 2H), 1.67-1.58 (m, 2H)

### Example 7: Synthesis of 3-(5-chloropyrimidin-2-yl)oxy-2-[3-(trifluoromethoxy) propylsulfanyl]benzonitrile - Compound A116.

### Step 1: Synthesis of 2-fluoro-3-(2-trimethylsilylethoxymethoxy)benzonitrile

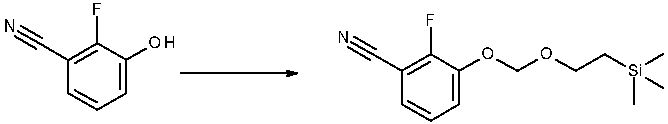

To a solution of 2-fluoro-3-hydroxy-benzonitrile (0.50g, 3.60 mmol) in DCM (18 mL) was added Et₃N (1.0 mL, 7.3 mL) and 2-(chloromethoxy)ethyl-trimethylsilane (0.70 mL, 4.0 mmol) and the reaction stirred at RT for 72 hours. The reaction was diluted with further DCM, washed with water and brine, dried over MgSO₄ and evaporated to dryness under reduced pressure. The crude product was purified by flash chromatography on silica gel using a gradient of 0-30% EtOAc/cyclohexane as eluent to give the desired product (0.50g, 51%).

¹H NMR (400 MHz, CDCl₃) δ 7.47 (dt, 1H), 7.25 - 7.19 (m, 1H), 7.16 (dd, 1H), 5.29 (s, 2H), 3.79 (dd, 2H), 0.99 - 0.91 (m, 2H), 0.02 - -0.02 (m, 9H).

### Step 2: Synthesis of 2-[3-(trifluoromethoxy)propylsulfanyl]-3-(2-trimethylsilyl ethoxymethoxy)benzonitrile

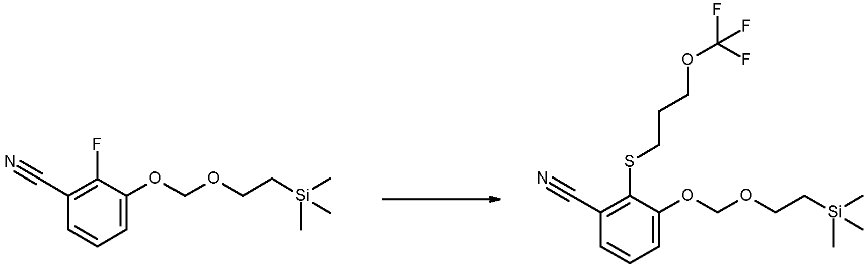

A mixture of 2-fluoro-3-(2-trimethylsilylethoxymethoxy)benzonitrile (0.25g, 0.93 mmol), sodium sulfide (0.103g, 1.87 mmol) in DMF (2.5 mL) under an N₂ atmosphere was heated at 50°C overnight. The reaction was allowed to cool to RT, then K₂CO₃ (0.129g, 0.93 mmol) and 1-bromo-3-(trifluoromethoxy)propane (0.213g, 1.03 mmol) were added and stirred at RT for 1.5 hours. The reaction was diluted with water and extracted with EtOAc (x 3). The combined organic extracts were washed with brine, dried over MgSO₄ and evaporated to dryness under reduced pressure. The crude product was purified by flash chromatography on silica gel using a gradient of 0-20% EtOAc/cyclohexane as eluent to give the desired product (0.286g, 61%).

¹H NMR (400 MHz, CDCl₃) δ 7.32 - 7.42 (m, 3H) 5.32 - 5.35 (m, 2H) 4.12 (t, 2H) 3.79 (dd, 2H) 3.07 (t, 2H) 1.91 (t, 2H) 0.95 (dd, 2H) -0.01 - 0.02 (m, 9H).

### Step 3: Synthesis of 3-hydroxy-2-[3-(trifluoromethoxy)propylsulfanyl]benzo nitrile

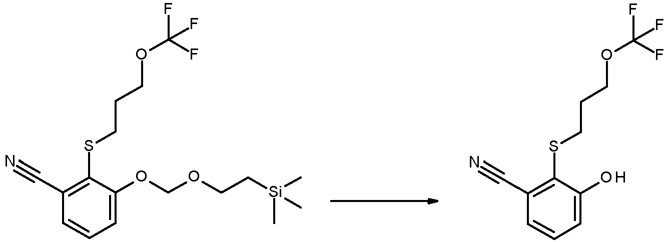

To a stirred solution of 2-[3-(trifluoromethoxy)propylsulfanyl]-3-(2-trimethylsilylethoxymethoxy)benzonitrile (0.286g, 0.70 mmol) in MeOH (2.1 mL) and THF (3.5 mL) was added 2M HCl (2.1 mL) and the reaction heated at 75°C for 2 hours. The reaction was allowed to cool to RT, diluted with water and extracted with EtOAc (x3). The combined organic extracts were dried over MgSO₄ and evaporated to dryness under reduced pressure. The crude product was purified by flash chromatography on silica gel using a gradient of 0-30% EtOAc/cyclohexane as eluent to give the desired product (0.154g, 79%).

¹H NMR (400 MHz, CDCl₃) δ 7.35 - 7.42 (m, 1H) 7.28 - 7.33 (m, 1H) 7.22 - 7.27 (m, 1H) 6.96 (s, 1H) 4.10 (t, 2H) 2.96 (t, 2H) 1.98 (quin, 2H).

### Step 4: Synthesis of 3-(5-chloropyrimidin-2-yl)oxy-2-[3-(trifluoromethoxy) propylsulfanyl]benzonitrile - Compound A116.

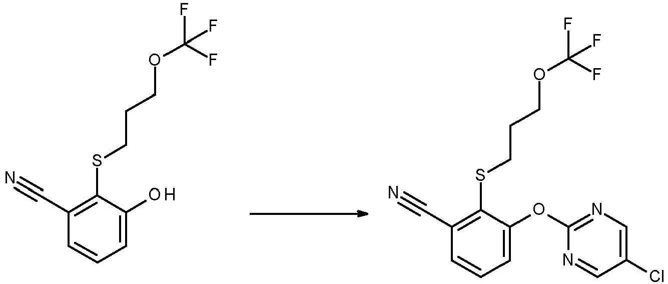

A mixture of 3-hydroxy-2-[3-(trifluoromethoxy)propylsulfanyl]benzonitrile (0.154g, 0.56 mmol), 5-chloro-2-methylsulfonyl-pyrimidine (0.128g, 0.67 mmol) and K₂CO₃ (0.092g, 0.67 mmol) in DMF (1.5 mL) was stirred at RT overnight. The reaction was diluted with water and extracted with EtOAc (x3). The combined organic extracts were dried over MgSO₄ and evaporated to dryness under reduced pressure. The crude product was purified by flash chromatography on silica gel using a gradient of 0-30% EtOAc/cyclohexane as eluent to give the desired product (0.175g, 81%).

¹H NMR (400 MHz, CDCl₃) δ 8.45 - 8.52 (m, 2H) 7.66 (dd, 1H) 7.53 (t, 1H) 7.43 - 7.49 (m, 1H) 4.07 (t, 2H) 3.05 (t, 2H) 1.89 (quin, 2H)

The following tables provide examples of specific compounds of the present invention.

**Table 1**

| 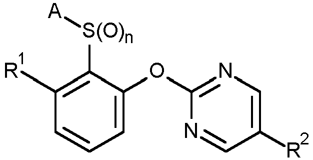 n = 0, R¹ = H and R² = CI | |
|---|---|
| **Compound** | **A** |
| 1.001 | prop-1-yl |
| 1.002 | but-1-yl |
| 1.003 | pent-1-yl |
| 1.004 | hex-1-yl |
| 1.005 | 3,3,3-trifluoroprop-1-yl |
| 1.006 | 4,4,4-trifluorobut-1-yl |
| 1.007 | 5,5,5-trifluoropent-1-yl |
| 1.008 | 6,6,6-trifluorohex-1-yl |
| 1.009 | *E*-CH₂CH=CHCF₃ |
| 1.010 | CH₂CH₂OCH₂CF₃ |
| 1.011 | CH₂CH₂OCF₃ |
| 1.012 | Heptafluoroprop-1-yl |
| 1.013 | nonafluorobut-1-yl |
| 1.014 | 3,3-difluoroprop-1-yl |
| 1.015 | 4,4-difluorobut-1-yl |
| 1.016 | 2,2,3,3-tetrafluoroprop-1-yl |
| 1.017 | 2,2,3,3,3-pentafluoroprop-1-yl |

Further compounds of the present invention are indicated via the additional tables provided below. Each table discloses various variations of n, R¹ and R² and should be interpreted as disclosing the A variants shown in Table 1 above. Thus, Table 2 discloses compounds 2.001 to 2.017, Table 3 discloses Compound 3.001 to 3.017 etc.

| **Table** | **n** | **R¹** | **R²** |
|---|---|---|---|
| 2 | 1 | H | Cl |
| 3 | 2 | H | Cl |
| 4 | 0 | F | Cl |
| 5 | 1 | F | Cl |
| 6 | 2 | F | Cl |
| 7 | 0 | Cl | Cl |
| 8 | 1 | Cl | Cl |
| 9 | 2 | Cl | Cl |
| 10 | 0 | Br | Cl |
| 11 | 1 | Br | Cl |
| 12 | 2 | Br | Cl |
| 13 | 0 | CN | Cl |
| 14 | 1 | CN | Cl |
| 15 | 2 | CN | Cl |
| 16 | 0 | H | Br |
| 17 | 1 | H | Br |
| 18 | 2 | H | Br |
| 19 | 0 | F | Br |
| 20 | 1 | F | Br |
| 21 | 2 | F | Br |
| 22 | 0 | Cl | Br |
| 23 | 1 | Cl | Br |
| 24 | 2 | Cl | Br |
| 25 | 0 | Br | Br |
| 26 | 1 | Br | Br |
| 27 | 2 | Br | Br |
| 28 | 0 | CN | Br |
| 29 | 1 | CN | Br |
| 30 | 2 | CN | Br |
| 31 | 0 | H | F |
| 32 | 1 | H | F |
| 33 | 2 | H | F |
| 34 | 0 | F | F |
| 35 | 1 | F | F |
| 36 | 2 | F | F |
| 37 | 0 | Cl | F |
| 38 | 1 | Cl | F |
| 39 | 2 | Cl | F |
| 40 | 0 | Br | F |
| 41 | 1 | Br | F |
| 42 | 2 | Br | F |
| 43 | 0 | CN | F |
| 44 | 1 | CN | F |
| 45 | 2 | CN | F |

**Table C1 - Herbicidal compounds of the present invention.**

| 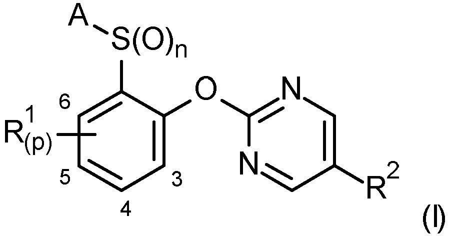 | | | | | |
|---|---|---|---|---|---|
| **Compound** | **n** | **A** | **R²** | **R¹** | **¹H NMR (400MHz, CDCl₃ unless stated)** |
| A1 | 0 | 3,3, 3-trifluoroprop-1-yl | Cl | - | 8.48 (s, 2H), 7.51 (dd, 1H), 7.42 - 7.36 (m, 1H), 7.33 - 7.27 (m, 1H), 7.21 (dd, 1H), 3.04 - 2.97 (m, 2H), 2.42 - 2.27 (m, 2H) |
| A2 | 1 | 3,3,3-trifluoroprop-1-yl | Cl | - | 8.52 (s, 2H), 7.92 (dd, 1H), 7.64 - 7.58 (m, 1H), 7.58 - 7.52 (m, 1H), 7.23 (dd, 1H), 3.28 (ddd, 1H), 3.06 (ddd, 1H), 2.75 - 2.58 (m, 1H), 2.26 - 2.10 (m, 1H) |
| A3 | 2 | 3,3,3-trifluoroprop-1-yl | Cl | - | 8.49 (s, 2H), 8.06 (dd, 1H), 7.79 (dt, 1H), 7.52 (dt, 1H), 7.38 (dd, 1H), 3.66 - 3.58 (m, 2H), 2.63 - 2.50 (m, 2H) |
| A4 | 0 | 4,4,4-trifluorobut-1-yl | Cl | - | 8.48 (s, 2H), 7.48 (dd, 1H), 7.40 - 7.31 (m, 1H), 7.31 - 7.24 (m, 1H), 7.19 (dd, 1H), 2.92 (t, 2H), 2.25 - 2.12 (m, 2H), 1.85 - 1.77 (m, 2H) |
| A5 | 1 | 4,4,4-trifluorobut-1-yl | Cl | - | 8.51 (s, 2H), 7.95 (dd, 1H), 7.70 - 7.48 (m, 2H), 7.21 (dd, 1H), 3.10 (br d, 1H), 3.01 - 2.85 (m, 1H), 2.37 - 1.77 (m, 4H) |
| A6 | 2 | 4,4,4-trifluorobut-1-yl | Cl | - | 8.49 (s, 2H), 8.07 (dd, 1H), 7.77 (dt, 1H), 7.51 (dt, 1H), 7.36 (dd, 1H), 3.48 (t, 2H), 2.36 - 2.21 (m, 2H), 2.07 - 1.96 (m, 2H) |
| A7 | 0 | 3,3,3-trifluoroprop-1-yl | Cl | 6-F | 8.48 (s, 2H), 7.43 (dt, 1H), 7.14 - 7.05 (m, 2H), 2.98 - 2.92 (m, 2H), 2.35 - 2.23 (m, 2H) |
| A8 | 1 | 3,3,3-trifluoroprop-1-yl | Cl | 6-F | 8.49 (s, 2H), 7.60 (dt, 1H), 7.18 (ddd, 1H), 7.10 - 7.05 (m, 1H), 3.73 - 3.64 (m, 1H), 3.34 - 3.25 (m, 1H), 2.66 - 2.54 (m, 2H) |
| A9 | 2 | 3,3,3-trifluoroprop-1-yl | Cl | 6-F | 8.49 (s, 2H), 7.74 (dt, 1H), 7.28 - 7.22 (m, 1H), 7.16 (td, 1H), 3.62 - 3.55 (m, 2H), 2.70 - 2.57 (m, 2H) |
| A10 | 0 | 3,3,3-trifluoroprop-1-yl | Cl | 6-Cl | 8.48 (s, 2H), 7.49 - 7.43 (m, 1H), 7.43 - 7.37 (m, 1H), 7.18 (dd, 1H), 3.01 - 2.95 (m, 2H), 2.36 - 2.23 (m, 2H) |
| A11 | 1 | 3,3,3-trifluoroprop-1-yl | Cl | 6-Cl | 8.46 (s, 2H), 7.57 - 7.50 (m, 1H), 7.40 (dd, 1H), 7.19 (dd, 1H), 3.68 (ddd, 1H), 3.33 (ddd, 1H), 2.71 - 2.55 (m, 2H) |
| A12 | 2 | 3,3,3-trifluoroprop-1-yl | Cl | 6-Cl | 8.48 (s, 2H), 7.68 - 7.62 (m, 1H), 7.55 (dd, 1H), 7.28 - 7.23 (m, 1H), 3.70 - 3.63 (m, 2H), 2.70 - 2.58 (m, 2H) |
| A13 | 0 | 3,3,3-trifluoroprop-1-yl | Cl | 6-CN | 8.49 (s, 2H), 7.69 (dd, 1H), 7.57 (t, 1H), 7.48 (dd, 1H), 3.16 - 3.06 (m, 2H), 2.44 - 2.28 (m, 2H) |
| A14 | 1 | 3,3, 3-trifluoroprop-1-yl | Cl | 6-CN | 8.51 (s, 2H), 7.82 (dd, 1H), 7.72 (t, 1H), 7.48 (dd, 1H), 3.52 (ddd, 1H), 3.35 (ddd, 1H), 2.83 - 2.65 (m, 1H), 2.55 (m, 1H) |
| A15 | 2 | 3,3,3-trifluoroprop-1-yl | Cl | 6-CN | 8.50 (s, 2H), 7.92 - 7.85 (m, 2H), 7.62 (dd, 1H), 3.76 - 3.70 (m, 2H), 2.75 - 2.63 (m, 2H) |
| A16 | 0 | benzyl | Cl | - | 8.46 (s, 2H), 7.39 - 7.35 (m, 1H), 7.35 - 7.28 (m, 1H), 7.28 7.16 (m, 7H), 4.06 (s, 2H) |
| A17 | 2 | CH₂(4-thiazolyl) | Cl | - | 8.68 (d, 1H), 8.50 (s, 2H), 7.79 (dd, 1H), 7.74 - 7.66 (m, 1H), 7.40 - 7.33 (m, 3H), 4.95 (s, 2H) |
| A18 | 0 | CH₂(4-thiazolyl) | Cl | - | 8.73 (d, 1H), 8.48 (s, 2H), 7.43 - 7.35 (m, 1H), 7.32 (s, 1H), 7.22 - 7.15 (m, 2H), 7.07 - 7.03 (m, 1H), 4.26 (s, 2H) |
| A19 | 2 | benzyl | Cl | - | 8.51 (s, 2H), 7.70 - 7.65 (m, 2H), 7.36 - 7.21 (m, 7H), 4.64 (s, 2H) |
| A20 | 2 | 1-butyl | Cl | - | 8.48 (s, 2H), 8.06 (dd, 1H), 7.74 (dt, 1H), 7.49 (dt, 1H), 7.33 (dd, 1H), 3.43 - 3.36 (m, 2H), 1.72 - 1.61 (m, 2H), 1.46 - 1.37 (m, 2H), 0.90 (t, 3H) |
| A21 | 0 | 1-butyl | Cl | - | 8.47 (s, 2H), 7.45 (dd, 1H), 7.32 - 7.22 (m, 2H), 7.20 - 7.12 (m, 1H), 2.89 - 2.84 (m, 2H), 1.60 - 1.50 (m, 2H), 1.44 - 1.33 (m, 2H), 0.88 (t, 3H) |
| A22 | 1 | 1-butyl | Cl | - | 8.51 (s, 2H), 7.96 (dd, 1H), 7.59 - 7.49 (m, 2H), 7.19 (dd, 1H), 3.03 (m, 1H), 2.86 (m, 1H), 1.91 - 1.69 (m, 1H), 1.63 - 1.52 (m, 1H), 1.52 - 1.30 (m, 2H), 0.90 (t, 3H) |
| A23 | 1 | benzyl | Cl | - | 8.52 (s, 2H), 7.54 - 7.47 (m, 2H), 7.36 - 7.29 (m, 1H), 7.28 - 7.19 (m, 4H), 7.09 - 7.03 (m, 2H), 4.31 (d, 1H), 4.02 (d, 1H) |
| A24 | 2 | 4,4,4-trifluorobut-1-yl | Cl | 6-Cl | 8.47 (s, 2H), 7.62 (t, 1H), 7.56 - 7.50 (m, 1H), 7.24 (dd, 1H), 3.52 (t, 2H), 2.37 - 2.25 (m, 2H), 2.14 - 2.04 (m, 2H) |
| A25 | 0 | 4,4,4-trifluorobut-1-yl | Cl | 6-Cl | 8.48 (s, 2H), 7.47 - 7.40 (m, 1H), 7.40 - 7.33 (m, 1H), 7.16 (dd, 1H), 2.91 (t, 2H), 2.29 - 2.16 (m, 2H), 1.77 - 1.66 (m, 2H) |
| A26 | 1 | 4,4,4-trifluorobut-1-yl | Cl | 6-Cl | 8.46 (s, 2H), 7.55 - 7.48 (m, 1H), 7.39 (dd, 1H), 7.18 (dd, 1H), 3.67 - 3.54 (m, 1H), 3.20 (td, 1H), 2.41 - 2.21 (m, 2H), 2.17 - 2.00 (m, 2H) |
| A27 | 0 | CH₂(4-Cl-C₆H₄) | Cl | - | 8.46 (s, 2H), 7.38 - 7.29 (m, 2H), 7.21 - 7.11 (m, 6H), 4.01 (s, 2H) |
| A28 | 0 | 4,4,4-trifluorobut-1-yl | Cl | 6-CN | 8.48 (s, 2H), 7.66 (dd, 1H), 7.53 (t, 1H), 7.48 - 7.42 (m, 1H), 3.05 - 2.99 (m, 2H), 2.35 - 2.17 (m, 2H), 1.81 - 1.72 (m, 2H) |
| A29 | 1 | 4,4,4-trifluorobut-1-yl | Cl | 6-CN | 8.50 (s, 2H), 7.80 (dd, 1H), 7.70 (t, 1H), 7.47 (dd, 1H), 3.50 - 3.41 (m, 1H), 3.23 - 3.12 (m, 1H), 2.40 - 2.25 (m, 2H), 2.19 - 2.06 (m, 2H) |
| A30 | 2 | 4,4,4-trifluorobut-1-yl | Cl | 6-CN | 8.49 (s, 2H), 7.90 - 7.83 (m, 2H), 7.61 (dd, 1H), 3.59 (t, 2H), 2.42 - 2.29 (m, 2H), 2.16 - 2.07 (m, 2H) |
| A31 | 1 | 2-pentyl | Cl | - | Characteristic signals: 8.52 - 8.48 (m, 2H), 7.96 - 7.87 (m, 1H), 7.59 - 7.48 (m, 2H), 7.22 - 7.16 (m, 1H) |
| A32 | 2 | 2-pentyl | Cl | - | 8.47 (s, 2H), 8.05 (dd, 1H), 7.73 (dt, 1H), 7.48 (dt, 1H), 7.32 (dd, 1H), 3.62 - 3.54 (m, 1H), 1.90 - 1.69 (m, 1H), 1.56 - 1.42 (m, 2H), 1.28 (m, 4H), 0.90 (t, 3H) |
| A33 | 0 | 2-pentyl | Cl | - | 8.47 (s, 2H), 7.52 (dd, 1H), 7.38 - 7.29 (m, 1H), 7.28 - 7.21 (m, 1H), 7.21 - 7.16 (m, 1H), 3.33 - 3.23 (m, 1H), 1.58 - 1.31 (m, 4H), 1.20 (d, 3H), 0.86 (s, 3H) |
| A34 | 2 | CH₂(4-Cl-C₆H₄) | CI | - | 8.51 (s, 2H), 7.74 - 7.67 (m, 2H), 7.42 - 7.31 (m, 2H), 7.25 - 7.16 (m, 4H), 4.62 (s, 2H) |
| A35 | 2 | CH₂(4-CN-C₆H₄) | Cl | - | 8.51 (s, 2H), 7.76 - 7.69 (m, 2H), 7.59 - 7.53 (m, 2H), 7.41 - 7.34 (m, 4H), 4.70 (s, 2H) |
| A36 | 0 | CH₂(4-CN-C₆H₄) | Cl | - | 8.47 (s, 2H), 7.54 - 7.48 (m, 2H), 7.38 - 7.23 (m, 4H), 7.23 - 7.11 (m, 2H), 4.06 (s, 2H) |
| A37 | 0 | *E*-CH₂CH=CHCl | Cl | - | 8.48 (s, 2H), 7.48 (dd, 1H), 7.41 - 7.34 (m, 1H), 7.30 - 7.23 (m, 1H), 7.20 (dd, 1H), 5.99 - 5.85 (m, 2H), 3.47 (dd, 2H) |
| A38 | 2 | *E*-CH₂CH=CHCl | Cl | - | 8.49 (s, 2H), 8.01 (dd, 1H), 7.79 - 7.72 (m, 1H), 7.50 (dt, 1H), 7.35 (dd, 1H), 6.23 (dt, 1H), 5.88 (dt, 1H), 4.13 (dd, 2H) |
| A39 | 0 | 5,5,5-trifluoropent-1-yl | Cl | 6-CN | 8.47 (s, 2H), 7.65 (dd, 1H), 7.52 (t, 1H), 7.49 - 7.42 (m, 1H), 2.97 (t, 2H), 2.13 - 1.99 (m, 2H), 1.67 - 1.56 (m, 4H) |
| A40 | 0 | 6,6,6-trifluorohex-1-yl | Cl | 6-CN | 8.48 (s, 2H), 7.65 (dd, 1H), 7.53 - 7.47 (m, 1H), 7.47 - 7.42 (m, 1H), 2.96 (t, 2H), 2.11 - 1.97 (m, 2H), 1.58 - 1.44 (m, 6H) |
| A41 | 0 | 2-methoxyethyl | Cl | - | 8.48 (s, 2H), 7.53 (d, 1H), 7.32 (t, 1H), 7.25 (t, 1H), 7.17 (d, 1H), 3.53 (t, 2H), 3.31 (s, 3H), 3.04 (t, 2H) |
| A42 | 2 | 2-methoxyethyl | Cl | - | 8.48 (s, 2H), 8.06 (d, 1H), 7.72 (d, 1H), 7.48 (t, 1H), 7.32 (d, 1H), 3.76 (t, 2H), 3.68 (t, 2H) |
| A43 | 2 | pent-1-yl | Cl | - | 8.48 (s, 2H), 8.06 (d, 1H), 7.73 (t, 1H), 7.48 (t, 1H), 7.33 (d, 1H), 3.48 (t, 2H), 1.72-1.65 (m, 2H), 1.39-1.25 (m, 4H), 0.87 (t, 3H) |
| A44 | 0 | pent-1-yl | Cl | - | 8.48 (s, 2H), 7.44 (d, 1H), 7.29-7.21 (m, 2H), 7.15 (d, 1H), 2.85 (t, 2H), 1.60-1.52 (m, 2H), 1.35-1.22 (m, 4H), 0.86 (t, 3H) |
| A45 | 0 | prop-2-ynyl | Cl | - | 8.48 (s, 2H), 7.64 (d, 1H), 7.36 (t, 1H), 7.27 (t, 1H), 7.20 (d, 1H), 3.58 (s, 2H), 2.20 (t, 1H) |
| A46 | 2 | prop-2-ynyl | Cl | - | 8.48 (s, 2H), 8.13 (d, 1H), 7.76 (t, 1H), 7.50 (t, 1H), 7.35 (d, 1H), 4.30 (s, 2H), 2.29 (t, 1H) |
| A47 | 0 | allyl | Cl | - | 8.48 (s, 2H), 7.47 (d, 1H), 7.31 (t, 1H), 7.25 (t, 1H), 7.15 (d, 1H), 5.85-5.75 (m, 1H), 5.12 (d, 1H), 5.04 (d, 1H), 3.51 (d, 2H) |
| A48 | 2 | allyl | Cl | - | 8.49 (s, 2H), 8.00 (d, 1H), 7.71 (t, 1H), 7.45 (t, 1H), 7.33 (d, 1H), 5.81-5.73 (m, 1H), 5.31-5.25 (m, 2H), 4.13 (d, 2H) |
| A49 | 0 | prop-1-yl | Cl | - | 8.48 (s, 2H), 7.45 (d, 1H), 7.31-7.22 (m, 2H), 7.15 (d, 1H), 2.84 (t, 2H), 1.62-1.55 (m, 2H), 0.96 (t, 3H) |
| A50 | 2 | prop-1-yl | Cl | - | 8.48 (s, 2H), 8.06 (d, 1H), 7,72 (t, 1H), 7.48 (t, 1H), 7.32 (d, 1H), 3.37 (t, 2H), 1.75-1.70 (m, 2H), 1.01 (t, 3H) |
| A51 | 2 | CH₂cyclopropyl | Cl | - | 8.48 (s, 2H), 8.11 (d, 1H), 7.73 (t, 1H), 7.48 (t, 1H), 7.31 (t, 1H), 3.33 (d, 2H), 1.05-0.97 (m, 1H), 0.55-0.49 (m, 2H), 0.23-0.20 (m, 2H) |
| A52 | 0 | 3-methylbut-2-enyl | Cl | - | 8.48 (s, 2H), 7.46 (d, 1H), 7.31 (t, 1H), 7.24 (t, 1H), 7.15 (d, 1H), 5.22 (t, 1H), 3.49 (d, 2H), 1.68 (s, 3H), 1.55 (s, 3H) |
| A53 | 0 | hex-1-yl | Cl | - | 8.48 (s, 2H), 7.45 (d, 1H), 7.30-7.21 (m, 2H), 7.15 (d, 1H), 2.85 (t, 2H), 1.60-1.52 (m, 2H), 1.38-1.30 (m, 2H), 1.29-1.18 (m, 4H), 0.86 (t, 3H) |
| A54 | 0 | CH₂CH₂OCF₃ | Cl | - | 8.48 (s, 2H), 7.55 (dd, 1H), 7.39 (t, 1H), 7.30 (t, 1H), 7.21 (dd, 1H), 4.02 (t, 2H), 3.13 (t, 2H) |
| A55 | 0 | CH₂CF₃ | Cl | - | 8.49 (s, 2H), 7.63 (d, 1H), 7.44 (t, 1H), 7.26 (t, 1H), 7.20 (d, 1H), 3.42 (q, 2H) |
| A56 | 0 | 3-methoxyprop-1-yl | Cl | - | 8.48 (s, 2H), 7.48 (d, 1H), 7.32-7.24 (m, 2H), 7.16 (d, 1H), 3.40 (t, 2H), 3.30 (s, 3H), 2.94 (t, 2H), 1.85-1.80 (m, 2H) |
| A57 | 0 | 2-(4-chloropyrazol-1-yl)ethyl | Cl | - | 8.49 (s, 2H), 7.50 (d, 1H), 7.40 (s, 1H), 7.38-7.34 (m, 3H), 7.25 (t, 1H), 7.20 (d, 1H), 4.20 (t, 2H), 3.25 (t, 2H) |
| A58 | 0 | CH₂cyclohexyl | Cl | - | 8.48 (s, 2H), 7.43 (d, 1H), 7.29-7.20 (m, 2H), 7.15 (d, 1H), 2.75 (d, 2H), 1.84-1.80 (m, 2H), 1.71-1.60 (m, 3H), 1.50-1.40 (m, 1H), 1.25-1.10 (m, 3H), 0.95-0.85 (m, 2H) |
| A59 | 0 | 3-fluoroprop-1-yl | Cl | - | 8.48 (s, 2H), 7.49 (d, 1H), 7.33 (t, 1H), 7.25 (t, 1H), 7.15 (d, 1H), 4.55 (t, 1H), 4.45 (t, 1H), 3.00 (t, 2H), 1.98-1.88 (m, 2H) |
| A60 | 0 | 2-fluoroethyl | Cl | - | 8.48 (s, 2H), 7.55 (d, 1H), 7.35 (t, 1H), 7.26 (t, 1H), 7.18 (d, 1H), 4.55 (t, 1H), 4.45 (t, 1H), 3.20-3.10 (m, 2H) |
| A61 | 0 | 2,2-difluoroethyl | Cl | - | 8.48 (s, 2H), 7.60 (d, 1H), 7.41 (t, 1H), 7.25 (t, 1H), 7.20 (d, 1H), 5.85 (dt, 1H), 3.19 (dq, 2H) |
| A62 | 0 | 2-methylallyl | Cl | - | 8.48 (s, 2H), 7.45 (d, 1H), 7.30 (t, 1H), 7.22 (t, 1H), 7.15 (d, 1H), 4.78 (d, 2H), 3.45 (s, 2H), 1.80 (s, 3H) |
| A63 | 0 | 4-methylpent-3-enyl | Cl | - | 8.48 (s, 2H), 7.46 (d, 1H), 7.30-7.20 (m, 2H), 7.15 (d, 1H), 5.10 (t, 1H), 2.85 (t, 2H), 2.25 (q, 2H), 1.66 (s, 3H), 1.55 (s, 3H) |
| A64 | 0 | 2-ethylbut-1-yl | Cl | - | 8.48 (s, 2H), 7.45 (d, 1H), 7.30-7.21 (m, 2H), 7.13 (d, 1H), 2.85 (d, 2H), 1.46-1.30 (m, 5H), 0.84 (t, 6H) |
| A65 | 0 | secbutyl | Cl | - | 8.48 (s, 2H), 7.54 (d, 1H), 7.33 (t, 1H), 7.23 (t, 1H), 7.18 (d, 1H), 3.27-3.20 (m, 1H), 1.62-1.55 (m, 1H), 1.50-1.43 (m, 1H), 1.20 (d, 3H), 0.92 (t, 3H) |
| A66 | 0 | (3-chlorophenyl)methyl | Cl | - | 8.47 (s, 2H), 7.36-7.30 (m, 2H), 7.20-7.12 (m, 5H), 7.06 (d, 1H), 4.01 (s, 2H) |
| A67 | 0 | (2-chlorophenyl)methyl | Cl | - | 8.45 (s, 2H), 7.38-7.30 (m, 3H), 7.20-7.05 (m, 5H), 4.14 (s, 2H) |
| A68 | 0 | 3,4,4-trifluorobut-3-enyl | Cl | - | 8.48 (s, 2H), 7.50 (d, 1H), 7.34 (t, 1H), 7.25 (t, 1H), 7.20 (d, 1H), 3.04 (t, 2H), 2.59-2.45 (m, 2H) |
| A69 | 0 | 2-methylpent-1-yl | Cl | - | 8.48 (s, 2H), 7.45 (d, 1H), 7.30-7.20 (m, 2H), 7.14 (d, 1H), 2.88 (dd, 1H), 2.66 (dd, 1H), 1.70-1.60 (m 1H), 1.40-1.10 (m, 4H), 0.94 (d, 3H), 0.85 (t, 3H) |
| A70 | 0 | Isopentyl | Cl | - | 8.48 (s, 2H), 7.45 (d, 1H), 7.31-7.22 (m, 2H), 7.15 (d, 1H), 2.86 (t, 2H), 1.7-1.62 (m, 1H), 1.48-1.40 (m, 2H), 0.85 (d, 6H) |
| A71 | 0 | but-3-enyl | Cl | - | 8.48 (s, 2H), 7.48 (d, 1H), 7.32-7.20 (m, 2H), 7.17 (d, 1H), 5.82-5.73 (m, 1H), 5.05-5.00 (m, 2H), 2.92 (t, 2H), 2.30 (q, 2H) |
| A72 | 0 | Isobutyl | Cl | - | 8.48 (s, 2H), 7.45 (d, 1H), 7.30-7.20 (m, 2H), 7.15 (d, 1H), 2.75 (d, 2H), 1.81-1.70 (m, 1H), 0.98 (d, 6H) |
| A73 | 2 | 3,4,4-trifluorobut-3-enyl | Cl | - | 8.49 (s, 2H), 8.08 (dd, 1H), 7.76 (t, 1H), 7.51 (t, 1H), 7.35 (d, 1H), 3.62 (t, 2H), 2.80-2.70 (m, 2H) |
| A74 | 2 | 2-methylpent-1-yl | Cl | - | 8.48 (s, 2H), 8.08 (d, 1H), 7.72 (t, 1H), 7.48 (t, 1H), 7.32 (d, 1H), 3.45 (dd, 1H), 3.23 (dd, 1H), 2.11-2.05 (m, 1H), 1.45-1.20 (m, 4H), 1.04 (d, 3H), 0.83 (t, 3H) |
| A75 | 2 | Isopentyl | Cl | - | 8.48 (s, 2H), 8.05 (dd, 1H), 7.74 (t, 1H), 7.48 (t, 1H), 7.32 (d, 1H), 3.41-3.36 (m, 2H), 1.70-1.61 (m, 1H), 1.60-1.52 (m, 2H), 0.90 (d, 6H) |
| A76 | 2 | Isobutyl | Cl | - | 8.48 (s, 2H), 8.06 (dd, 1H), 7.71 (t, 1H), 7.49 (t, 1H), 7.31 (d, 1H), 3.31 (d, 2H), 2.25-2.18 (m, 1H), 1.05 (d, 6H) |
| A77 | 2 | Hex-1-yl | Cl | - | 8.48 (s, 2H), 8.05 (dd, 1H), 7.72 (t, 1H), 7.48 (t, 1H), 7.33 (d, 1H), 3.41-3.35 (m, 2H), 1.70-1.65 (m, 2H), 1.40-1.35 (m, 2H), 1.25-1.20 (m, 4H), 0.85 (t, 3H) |
| A78 | 2 | 2,2,2-trifluoroethyl | Cl | - | 8.49 (s, 2H), 8.11 (dd, 1H), 7.80 (t, 1H), 7.52 (t, 1H), 7.48 (d, 1H), 4.26 (q, 2H) |
| A79 | 2 | 3-methoxyprop-1-yl | Cl | - | 8.48 (s, 2H), 8.05 (dd, 1H), 7.72 (t, 1H), 7.49 (t, 1H), 7.31 (d, 1H), 3.51-3.45 (m, 2H), 3.42 (t, 2H), 3.29 (s, 3H), 1.99-1.92 (m, 2H) |
| A80 | 2 | 2-(4-chloropyrazol-1-yl)ethyl | Cl | - | 8.48 (s, 2H), 7.90 (dd, 1H), 7.70 (t, 1H), 7.44 (t, 1H), 7.35 (s, 1H), 7.30 (d, 1H), 7.21 (s, 1H), 4.52 (t, 2H), 4.01 (t, 2H) |
| A81 | 2 | CH2cyclohexyl | Cl | - | 8.48 (s, 2H), 8.07 (dd, 1H), 7.73 (t, 1H), 7.48 (t, 1H), 7.31 (d, 1H), 3.30 (d, 2H), 2.02-1.91 (m, 1H), 1.88-1.80 (m, 2H), 1.70-1.58 (m, 4H), 1.28-1.02 (m, 4H) |
| A82 | 2 | 3-fluoroprop-1-yl | Cl | - | 8.49 (s, 2H), 8.08 (dd, 1H), 7.73 (t, 1H), 7.50 (t, 1H), 7.33 (d, 1H), 4.58 (t, 1H), 4.48 (t, 1H), 3.54 (t, 2H), 2.20-2.05 (m, 2H) |
| A83 | 2 | 2-fluoroethyl | Cl | - | 8.48 (s, 2H), 8.08 (dd, 1H), 7.75 (t, 1H), 7.50 (t, 1H), 7.35 (d, 1H), 4.87 (t, 1H), 4.75 (t, 1H), 3.85 (t, 1H), 3.81 (t, 1H) |
| A84 | 2 | 2,2-difluoroethyl | Cl | - | 8.49 (s, 2H), 8.05 (dd, 1H), 7.89 (t, 1H), 7.51 (t, 1H), 7.38 (d, 1H), 6.22 (tt, 1H), 4.01 (dt, 2H) |
| A85 | 2 | 2-methylallyl | Cl | - | 8.48 (s, 2H), 8.08 (dd, 1H), 7.75 (t, 1H), 7.51 (t, 1H), 7.34 (d, 1H), 3.85 (d, 1H), 3.40 (d, 1H), 2.85 (d, 1H), 2.53 (d, 1H), 1.52 (s, 3H) |
| A86 | 2 | 4-methylpent-3-enyl | Cl | - | 8.48 (s, 2H), 8.04 (dd, 1H), 7.73 (t, 1H), 7.48 (t, 1H), 7.33 (d, 1H), 3.67 (t, 2H), 2.95 (t, 2H), 2.66-2.58 (m, 1H), 1.12 (s, 3H), 1.10 (s, 3H) |
| A87 | 2 | 2-ethylbut-1-yl | Cl | - | 8.48 (s, 2H), 8.07 (dd, 1H), 7.72 (t, 1H), 7.48 (t, 1H), 7.33 (d, 1H), 3.35 (d, 2H), 1.90-1.85 (m, 1H), 1.48-1.40 (m, 4H), 0.84 (t, 6H) |
| A88 | 2 | sec-butyl | Cl | - | 8.48 (s, 2H), 8.04 (dd, 1H), 7.73 (t, 1H), 7.48 (t, 1H), 7.32 (d, 1H), 3.55-3.45 (m, 1H), 1.98-1.85 (m, 1H), 1.60-1.50 (m, 1H) 1.30 (d, 3H), 1.01 (t, 3H) |
| A89 | 2 | (3-chlorophenyl)methyl | Cl | - | 8.51 (s, 2H), 7.75-7.65 (m, 2H), 7.37-7.32 (m, 2H), 7.28-7.26 (m, 2H), 7.20-7.12 (m 2H), 4.62 (s, 2H) |
| A90 | 2 | (2-chlorophenyl)methyl | Cl | - | 8.49 (s, 2H), 7.77-7.68 (m, 2H), 7.38-7.30 (m, 4H), 7.27-7.15 (m, 2H), 4.85 (s, 2H) |
| A91 | 2 | 5,5,5-trifluoropent-1-yl | Cl | 2-CN | 8.48 (s, 2H), 7.90 - 7.81 (m, 2H), 7.59 (dd, 1H), 3.56 - 3.49 (m, 2H), 2.22 - 2.07 (m, 2H), 1.93 (quin, 2H), 1.81 - 1.70 (m, 2H) |
| A92 | 2 | 6,6,6-trifluorohex-1-yl | Cl | 2-CN | 8.51 - 8.45 (m, 2H), 7.91 - 7.80 (m, 2H), 7.58 (dd, , 1H), 3.55 - 3.46 (m, 2H), 2.16 - 1.99 (m, 2H), 1.93 - 1.78 (m, 2H), 1.66 - 1.50 (m, 4H) |
| A93 | 0 | 1,1,2,2,3,3,3-heptafluoroprop-1-yl | Cl | 2-CN | 8.48 (s, 2H), 7.70-7.77 (m, 2H), 7.55-7.57 (dd, 1H) |
| A94 | 0 | 3,3-difluoroprop-1-yl | Cl | 2-Cl | 8.48 (s, 2H), 7.44 (d, 1H), 7.38 (t, 1H), 7.17 (d, 1H), 5.93 (tt, 1H), 2.93 (t, 2H), 2.06-1.91 (m, 2H) |
| A95 | 1 | 3,3-difluoroprop-1-yl | Cl | 2-Cl | 8.46 (s, 2H), 7.51 (t, 1H), 7.38 (d, 1H), 7.17 (d, 1H), 6.00 (tt, 1H), 3.65-3.57 (m, 1H), 3.33-3.23 (m, 1H), 2.42-2.38 (m, 2H) |
| A96 | 2 | 3,3-difluoroprop-1-yl | Cl | 2-Cl | 8.48 (s, 2H), 7.61 (t, 1H), 7.52 (d, 1H), 7.22 (d, 1H), 6.00 (tt, 1H), 3.60 (t, 2H), 2.42-2.28 (m, 2H) |
| A97 | 2 | 3,3-difluoroprop-1-yl | Cl | 2-CN | 8.48 (s, 2H), 7.90-7.81 (m, 2H), 7.59 (d, 1H), 6.03 (tt, 1H), 3.68 (t, 2H), 2.48-2.31 (m, 2H) |
| A98 | 1 | 3,3-difluoroprop-1-yl | Cl | 2-CN | 8.49 (s, 2H), 7.79 (d, 1H), 7.69 (t, 1H), 7.46 (d, 1H), 6.01 (tt, 1H), 3.51-3.41 (m, 1H), 3.34-3.23 (m, 1H), 2.53-2.21 (m, 2H) |
| A99 | 0 | 3,3-difluoroprop-1-yl | Cl | 2-CN | 8.48 (s, 2H), 7.68 (d, 1H), 7.56 (t, 1H), 7.48 (d, 1H), 5.94 (tt, 1H), 3.06 (t, 2H), 2.12-1.98 (m, 2H) |
| A100 | 0 | 4,4-difluorobut-1-yl | Cl | 2-CN | 8.48 (s, 2H), 7.63 (d, 1H), 7.52 (t, 1H), 7.42 (d, 1H), 5.79 (tt, 1H), 2.99 (t, 2H), 2.01-1.85 (m, 2H), 1.71-1.61 (m, 2H) |
| A101 | 1 | 4,4-difluorobut-1-yl | Cl | 2-CN | 8.49 (s, 2H), 7.78 (d, 1H), 7.67 (t, 1H), 7.43 (d, 1H), 5.84 (tt, 1H), 3.49-3.39 (m, 1H), 3.22-3.12 (m, 1H), 2.12-1.91 (m, 4H) |
| A102 | 2 | 4,4-difluorobut-1-yl | Cl | 2-CN | 8.48 (s, 2H), 7.89-7.78 (m, 2H), 7.57 (d, 1H), 5.86 (tt, 1H), 3.54 (t, 2H), 2.12-1.98 (m, 4H) |
| A103 | 2 | 4,4-difluorobut-1-yl | Cl | 2-Cl | 8.48 (s, 2H), 7.60 (t, 1H), 7.52 (d, 1H), 7.21 (d, 1H), 5.81 (tt, 1H), 3.49 (t, 2H), 2.08-1.92 (m, 4H) |
| A104 | 1 | 4,4-difluorobut-1-yl | Cl | 2-Cl | 8.45 (s, 2H), 7.50 (t, 1H), 7.38 (d, 1H), 7.16 (d, 1H), 5.84 (tt, 1H), 3.61-3.52 (m, 1H), 3.24-3.15 (m, 1H), 2.11-1.89 (m, 4H) |
| A105 | 0 | 4,4-difluorobut-1-yl | Cl | 2-Cl | 8.47 (s, 2H), 7.41 (d, 1H), 7.33 (t, 1H), 7.13 (d, 1H), 5.78 (tt, 1H), 2.89 (t, 2H), 2.00-1.85 (m, 2H), 1.67-1.58 (m, 2H) |
| A106 | 0 | 2-trifluoromethoxyethyl | Cl | 2-CN | 8.49 (s, 2H), 7.65 (d, 1H), 7.53 (t, 1H), 7.45 (d, 1H), 4.05 (t, 2H), 3.22 (t, 2H) |
| A107 | 0 | 2-(2,2,2-trifluoroethoxy)ethyl | Cl | 2-CN | 8.51 - 8.45 (m, 2H), 7.65 (dd, 1H), 7.53 (t, 1H), 7.48 - 7.42 (m, 1H), 3.89 - 3.69 (m, 4H), 3.14 (t, 2H) |
| A108 | 2 | 2-(2,2,2-trifluoroethoxy)ethyl | Cl | 2-CN | 8.52 - 8.46 (m, 2H), 7.85 - 7.77 (m, 2H), 7.56 (dd, 1H), 4.17 - 4.07 (m, 2H), 3.84 - 3.79 (m, 2H), 3.75 (q, 2H) |
| A109 | 2 | 1,1,2,2,3,3,3-heptafluoroprop-1-yl | Cl | 2-CN | 8.48 (s, 2H), 7.70-7.78 (m, 2H), 7.55-7.57 (dd, 1H) |
| A110 | 0 | 2,2,3,3,3-pentafluoroprop-1-yl | Cl | 2-CN | 8.48 (s, 2H), 7.65-7.67 (d, 1H), 7.54-7.58 (t, 1H), 7.46-7.47 (d, 1H), 3.52 (t, 2H) |
| A111 | 0 | 2,2,3,3-tetrafluoroprop-1-yl | Cl | 2-CN | 8.47 (s, 2H), 7.65-7.66 (d, 1H), 7.54-7.58 (t, 1H), 7.46-7.48 (d, 1H), 6.06 (t, 1H), 3.46 (t, 2H) |
| A112 | 2 | 4,4,4-trifluorobut-1-yl | OC H₃ | 2-CN | 8.19 - 8.23 (m, 2H) 7.78 - 7.85 (m, 2H) 7.58 (dd, 1H), 3.90 (s, 3H) 3.61 (t, 2H) 2.28 - 2.42 (m, 2H) 2.07 - 2.19 (m, 2H) |
| A113 | 0 | 4,4,4-trifluorobut-1-yl | OC H₃ | 2-CN | 8.21 (s, 2H) 7.62 (dd, 1H) 7.47 - 7.52 (m, 1H) 7.41 - 7.47 (m, 1H) 3.89 (s, 3H) 3.04 (t, 2H) 2.16 - 2.29 (m, 2H) 1.72 - 1.81 (m, 2H) |
| A114 | 0 | 4,4,4-trifluorobut-1-yl | CH₃ | 2-CN | 8.36 (d, 2H) 7.63 (dd, 1H) 7.48 - 7.54 (m, 1H) 7.42 - 7.48 (m, 1H) 3.03 (t, 2H) 2.29 (s, 3H) 2.15 - 2.27 (m, 2H) 1.70 - 1.81 (m, 2H) |
| A115 | 2 | 4,4,4-trifluorobut-1-yl | CH₃ | 2-CN | 8.37 (d, 2H) 7.80 - 7.86 (m, 2H) 7.59 (dd, 1H) 3.61 (t, 2 H) 2.35 (dt, 2H) 2.29 (s, 3H) 2.07 - 2.18 (m, 2H) |
| A116 | 0 | 3-trifluoromethoxy-prop-1-yl | Cl | 2-CN | 8.45 - 8.52 (m, 2H) 7.66 (dd, 1H) 7.53 (t, 1H) 7.43 - 7.49 (m, 1H) 4.07 (t, 2H) 3.05 (t, 2H) 1.89 (quin, 2H) |
| A117 | 2 | 3-trifluoromethoxy-prop-1-yl | Cl | 2-CN | 8.47- 8.51 (m, 2H) 7.82 - 7.90 (m, 2H) 7.60 (dd, 1H) 4.09-4.15 (m, 2H) 3.59-3.66 (m, 2H) 2.19 - 2.33 (m, 2H) |
| A118 | 2 | 4,4,4-trifluorobut-1-yl | Br | 2-CN | 8.56 - 8.59 (m, 2H) 7.82 - 7.90 (m, 2H) 7.60 (dd, 1H) 3.58 (t, 2H) 2.29 - 2.42 (m, 2H) 2.06 - 2.19 (m, 2H) |
| A119 | 0 | 4,4,4-trifluorobut-1-yl | Br | 2-CN | 8.53-8.59 (m, 2H) 7.66 (dd, 1H) 7.53 (t, 1H) 7.44 - 7.49 (m, 1H) 3.03 (t, 2H) 2.17- 2.30 (m, 2H) 1.72-1.81 (m, 2H) |
| A120 | 2 | 5,5,5-trifluoropent-1-yl | Br | 2-CN | 8.57 (s, 2H), 7.88-7.81 (m, 2H), 7.58 (dd, 1H), 3.54-3.50 (m, 2H), 2.17-2.10 (m, 2H), 1.95-1.91 (m, 2H), 1.78-1.70 (m, 2H) |
| A121 | 0 | 5,5,5-trifluoropent-1-yl | Br | 2-CN | 8.56 (s, 2H), 7.65 (dd, 1H), 7.51 (t, 1H), 7.49-7.43 (m, 1H), 2.97 (t, 2H), 2.08-2.03 (m, 2H), 1.65-1.58 (m, 4H) |

### Biological Examples

### Test 1

Seeds of a variety of test species are sown in standard soil in pots *(Lolium perenne* (LOLPE), *Solanum nigrum* (SOLNI), *Amaranthus retoflexus* (AMARE), *Setaria faberi* (SETFA), *Echinochloa crus-galli* (ECHCG) and *Ipomoea hederacea* (IPOHE)). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 250 g/ha unless otherwise stated. The test plants are then grown in a glasshouse under controlled conditions in a glasshouse (at 24/16°C, day/night; 14 hours light; 65 % humidity) and watered twice daily. After 13 days for pre and post-emergence, the test is evaluated for the percentage damage caused to the plant. The biological activities (% phytotoxicity) are shown in Tables B1 and B2 below using a five-point scale (*5* = 81 to 100%; *4* = 61 to 80%; *3* = 41 to 60%; *2* = 21 to 40%; *1* = 0 to 20%).

**TABLE B1. Post-emergence application.**

| **Compound** | **AMARE** | **SOLNI** | **SETFA** | **LOLPE** | **ECHCG** | **IPOHE** |
|---|---|---|---|---|---|---|
| A1 | 5 | 4 | 4 | 4 | 4 | 4 |
| A2 | 5 | 5 | 4 | 4 | 4 | 4 |
| A3 | 5 | 5 | 4 | 4 | 4 | 3 |
| A4 | 5 | 5 | 5 | 4 | 4 | 4 |
| A5 | 5 | 5 | 5 | 5 | 5 | 4 |
| A6 | 5 | 5 | 4 | 2 | 3 | 3 |
| A7 | 5 | 5 | 5 | 2 | 4 | 2 |
| A8 | 5 | 4 | 3 | 1 | 1 | 2 |
| A9 | 5 | 5 | 4 | 3 | 3 | 3 |
| A10 | 5 | 5 | 5 | 5 | 5 | 4 |
| A11 | 5 | 5 | 5 | 4 | 5 | 5 |
| A12 | 5 | 5 | 5 | 5 | 5 | 4 |
| A13 | 5 | 5 | 5 | 5 | 5 | 4 |
| A14 | 5 | 5 | 5 | 4 | 5 | 5 |
| A15 | 5 | 5 | 5 | 4 | 5 | 5 |
| A16 | 4 | 3 | 2 | 1 | 1 | 1 |
| A20 | 2 | NT | 3 | NT | 2 | 1 |
| A21 | 4 | NT | 4 | NT | 4 | 3 |
| A22 | 3 | NT | 4 | NT | 4 | 2 |
| A24 | 5 | 5 | 5 | 5 | 5 | 5 |
| A25 | 5 | 5 | 5 | 5 | 5 | 3 |
| A26 | 5 | 5 | 5 | 5 | 5 | 5 |
| A27 | 5 | 3 | 2 | 1 | 2 | 2 |
| A28 | 5 | 5 | 5 | 5 | 5 | 5 |
| A29 | 5 | 5 | 5 | 5 | 5 | 5 |
| A30 | 5 | 5 | 5 | 5 | 5 | 4 |
| A33 | 3 | NT | 2 | NT | 2 | 1 |
| A36 | 5 | NT | 4 | NT | 3 | 3 |
| A37 | 4 | NT | 2 | NT | 2 | 2 |
| A38 | 2 | NT | 1 | NT | 1 | 3 |
| A39 | 5 | NT | 5 | NT | 5 | 3 |
| A40 | 5 | NT | 4 | NT | 4 | 3 |
| A41 | 3 | NT | 2 | NT | 3 | 3 |
| A42 | 2 | NT | 1 | NT | 1 | 1 |
| A43 | 3 | NT | 2 | NT | 2 | 1 |
| A44 | 5 | NT | 2 | NT | 4 | 1 |
| A45 | 3 | NT | 2 | NT | 3 | 1 |
| A46 | 1 | NT | 1 | NT | 1 | 1 |
| A48 | 2 | NT | 1 | NT | 1 | 1 |
| A49 | 4 | NT | 4 | NT | 4 | 4 |
| A50 | 4 | NT | 1 | NT | 1 | 2 |
| A51 | 3 | NT | 1 | NT | 1 | 1 |
| A52 | 3 | NT | 3 | NT | 4 | 1 |
| A53 | 4 | NT | 1 | NT | 1 | 1 |
| A55 | 5 | NT | 4 | NT | 4 | 4 |
| A56 | 2 | NT | 1 | NT | 1 | 1 |
| A57 | 5 | NT | 4 | NT | 4 | 2 |
| A59 | 5 | NT | 2 | NT | 4 | 2 |
| A60 | 3 | NT | 1 | NT | 1 | 2 |
| A61 | 5 | NT | 2 | NT | 2 | 2 |
| A62 | 1 | NT | 3 | NT | 4 | 1 |
| A64 | 2 | NT | 2 | NT | 1 | 2 |
| A65 | 1 | NT | 3 | NT | 4 | 3 |
| A66 | 4 | NT | 3 | NT | 4 | 2 |
| A68 | 5 | NT | 4 | NT | 5 | 5 |
| A69 | 4 | NT | 2 | NT | 4 | 1 |
| A70 | 2 | NT | 4 | NT | 5 | 4 |
| A71 | 5 | NT | 3 | NT | 4 | 4 |
| A72 | 3 | NT | 2 | NT | 3 | 2 |
| A73 | 4 | NT | 3 | NT | 3 | 1 |
| A74 | 3 | NT | 1 | NT | 1 | 1 |
| A75 | 2 | NT | 2 | NT | 3 | 2 |
| A76 | 1 | NT | 1 | NT | 2 | 2 |
| A77 | 2 | NT | 1 | NT | 1 | 1 |
| A82 | 3 | NT | 1 | NT | 1 | 3 |
| A87 | 1 | NT | 1 | NT | 1 | 1 |
| A88 | 2 | NT | 1 | NT | 1 | 1 |
| A89 | 3 | NT | 1 | NT | 1 | 1 |
| A91 | 5 | NT | 4 | NT | 5 | 4 |
| A92 | 5 | NT | 2 | NT | 3 | 2 |
| A94 | 5 | NT | 5 | NT | 5 | 3 |
| A95 | 4 | NT | 1 | NT | 2 | 4 |
| A96 | 5 | NT | 5 | NT | 5 | 5 |
| A97 | 5 | NT | 2 | NT | 4 | 4 |
| A98 | 5 | NT | 4 | NT | 4 | 4 |
| A99 | 5 | NT | 4 | NT | 5 | 4 |
| A100 | 5 | NT | 4 | NT | 5 | 5 |
| A101 | 5 | NT | 4 | NT | 4 | 4 |
| A102 | 5 | NT | 4 | NT | 4 | 4 |
| A103 | 4 | NT | 1 | NT | 1 | 2 |
| A104 | 5 | NT | 5 | NT | 5 | 5 |
| A105 | 5 | NT | 5 | NT | 5 | 4 |
| A106 | 5 | NT | 4 | NT | 5 | 3 |
| A107 | 5 | NT | 4 | NT | 5 | 2 |
| A108 | 4 | NT | 1 | NT | 1 | 2 |
| A112 | 2 | NT | 1 | NT | 1 | 2 |
| A113 | 5 | NT | 2 | NT | 3 | 3 |
| A114 | 3 | NT | 3 | NT | 4 | 4 |
| A115 | 4 | NT | 3 | NT | 4 | 4 |
| A116 | 5 | NT | 4 | NT | 4 | 4 |
| A117 | 5 | NT | 4 | NT | 4 | 3 |
| A118 | 5 | NT | 4 | NT | 4 | 4 |
| A119 | 5 | NT | 4 | NT | 4 | 4 |
| A120 | 4 | NT | 3 | NT | 3 | 4 |
| A121 | 5 | NT | 4 | NT | 3 | 3 |

**TABLE B2. Pre-emergence application.**

| **Compound** | **AMARE** | **SOLNI** | **SETFA** | **LOLPE** | **ECHCG** | **IPOHE** |
|---|---|---|---|---|---|---|
| A1 | 5 | 4 | 5 | 3 | 4 | 3 |
| A2 | 5 | 5 | 5 | 3 | 5 | 2 |
| A3 | 5 | 4 | 5 | 4 | 5 | 3 |
| A4 | 5 | 4 | 5 | 4 | 5 | 3 |
| A5 | 5 | 5 | 5 | 5 | 5 | 5 |
| A6 | 5 | 3 | 5 | 2 | 4 | 2 |
| A7 | 5 | 4 | 5 | 4 | 5 | 2 |
| A8 | 5 | 4 | 4 | 2 | 2 | 2 |
| A9 | 5 | 5 | 5 | 4 | 4 | 4 |
| A10 | 5 | 4 | 5 | 4 | 5 | 3 |
| A11 | 5 | 5 | 5 | 5 | 5 | 5 |
| A12 | 5 | 5 | 5 | 5 | 5 | 5 |
| A13 | 5 | 5 | 5 | 5 | 5 | 4 |
| A14 | 5 | 5 | 5 | 3 | 5 | 5 |
| A15 | 5 | 5 | 5 | 5 | 5 | 5 |
| A16 | 4 | 1 | 2 | 1 | 2 | 1 |
| A20 | 3 | NT | 4 | NT | 4 | 1 |
| A21 | 4 | NT | 5 | NT | 5 | 2 |
| A22 | 4 | NT | 5 | NT | 5 | 5 |
| A24 | 5 | 5 | 5 | 4 | 5 | 5 |
| A25 | 5 | 5 | 5 | 5 | 5 | 1 |
| A26 | 5 | 5 | 5 | 5 | 5 | 5 |
| A27 | 5 | 2 | 4 | 1 | 4 | 1 |
| A28 | 5 | 5 | 5 | 5 | 5 | 5 |
| A29 | 5 | 5 | 5 | 5 | 5 | 5 |
| A30 | 5 | 5 | 5 | 5 | 5 | 5 |
| A33 | 1 | NT | 3 | NT | 1 | 1 |
| A36 | 5 | NT | 5 | NT | 4 | 2 |
| A37 | 4 | NT | 3 | NT | 4 | 1 |
| A38 | 2 | NT | 1 | NT | 1 | 1 |
| A39 | 5 | NT | 5 | NT | 5 | 2 |
| A40 | 5 | NT | 5 | NT | 4 | 1 |
| A41 | 1 | NT | 2 | NT | 2 | 2 |
| A42 | 1 | NT | 1 | NT | 1 | 1 |
| A43 | 5 | NT | 4 | NT | 4 | 1 |
| A44 | 5 | NT | 5 | NT | 5 | 1 |
| A45 | 3 | NT | 3 | NT | 3 | 1 |
| A46 | 1 | NT | 1 | NT | 1 | 1 |
| A48 | 1 | NT | 1 | NT | 1 | 1 |
| A49 | 5 | NT | 5 | NT | 5 | 3 |
| A50 | 5 | NT | 5 | NT | 4 | 2 |
| A51 | 4 | NT | 2 | NT | 1 | 1 |
| A52 | 1 | NT | 1 | NT | 2 | 1 |
| A53 | 3 | NT | 3 | NT | 2 | 1 |
| A55 | 5 | NT | 5 | NT | 5 | 2 |
| A56 | 2 | NT | 2 | NT | 1 | 2 |
| A57 | 4 | NT | 4 | NT | 3 | 1 |
| A59 | 5 | NT | 4 | NT | 5 | 2 |
| A60 | 4 | NT | 3 | NT | 2 | 1 |
| A61 | 5 | NT | 4 | NT | 3 | 2 |
| A62 | 1 | NT | 3 | NT | 3 | 1 |
| A64 | 1 | NT | 1 | NT | 1 | 1 |
| A65 | 3 | NT | 4 | NT | 4 | 1 |
| A66 | 1 | NT | 2 | NT | 2 | 1 |
| A68 | 5 | NT | 5 | NT | 5 | 4 |
| A69 | 1 | NT | 4 | NT | 3 | 1 |
| A70 | 2 | NT | 5 | NT | 5 | 1 |
| A71 | 5 | NT | 5 | NT | 5 | 1 |
| A72 | 4 | NT | 4 | NT | 4 | 1 |
| A73 | 5 | NT | 4 | NT | 5 | 1 |
| A74 | 1 | NT | 1 | NT | 1 | 1 |
| A75 | 5 | NT | 4 | NT | 4 | 1 |
| A76 | 5 | NT | 4 | NT | 3 | 2 |
| A77 | 4 | NT | 1 | NT | 3 | 1 |
| A78 | 3 | NT | 1 | NT | 1 | 1 |
| A82 | 5 | NT | 3 | NT | 3 | 1 |
| A87 | 1 | NT | 1 | NT | 1 | 1 |
| A88 | 5 | NT | 5 | NT | 1 | 2 |
| A89 | 1 | NT | 1 | NT | 1 | 1 |
| A91 | 5 | NT | 5 | NT | 5 | 5 |
| A92 | 5 | NT | 4 | NT | 4 | 1 |
| A94 | 5 | NT | 5 | NT | 5 | 3 |
| A95 | 5 | NT | 5 | NT | 5 | 5 |
| A96 | 5 | NT | 5 | NT | 5 | 5 |
| A97 | 5 | NT | 4 | NT | 4 | 5 |
| A98 | 5 | NT | 5 | NT | 5 | 5 |
| A99 | 5 | NT | 5 | NT | 5 | 5 |
| A100 | 5 | NT | 5 | NT | 5 | 5 |
| A101 | 5 | NT | 5 | NT | 5 | 5 |
| A102 | 5 | NT | 5 | NT | 5 | 5 |
| A103 | 4 | NT | 2 | NT | 2 | 4 |
| A104 | 5 | NT | 5 | NT | 5 | 5 |
| A105 | 5 | NT | 5 | NT | 5 | 4 |
| A106 | 5 | NT | 5 | NT | 5 | 2 |
| A107 | 5 | NT | 5 | NT | 5 | 3 |
| A108 | 5 | NT | 1 | NT | 1 | 1 |
| A112 | 3 | NT | 1 | NT | 1 | 3 |
| A113 | 5 | NT | 2 | NT | 4 | 3 |
| A114 | 5 | NT | 4 | NT | 4 | 4 |
| A115 | 5 | NT | 3 | NT | 4 | 5 |
| A116 | 5 | NT | 5 | NT | 5 | 5 |
| A117 | 5 | NT | 5 | NT | 4 | 5 |
| A118 | 5 | NT | 5 | NT | 5 | 5 |
| A119 | 5 | NT | 5 | NT | 5 | 5 |
| A120 | 4 | NT | 4 | NT | 3 | 5 |
| A121 | 5 | NT | 5 | NT | 3 | 2 |

| | | | | | | |
|---|---|---|---|---|---|---|
| NT = Not tested. | | | | | | |

## Claims

1. A compound of Formula (I): 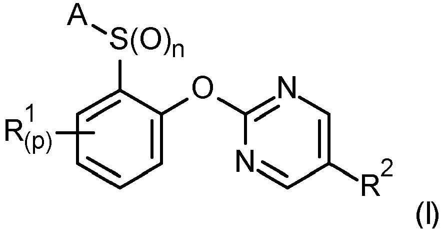 wherein
A is selected from the group consisting of C₃-C₈ alkyl, C₃-C₈ alkenyl, C₃-C₈ alkynyl, C₃-C₈ haloalkyl, C₃-C₈ haloalkenyl, C₃-C₈ haloalkynyl, C₁-C₄alkoxy-C₁-C₃alkyl-, C₁-C₄haloalkoxy-C₁-C₃alkyl-, C₁-C₄alkoxy-C₁-C₃haloalkyl- and - (CH₂)ₘR³;
R¹ is independently selected from the group consisting of halogen, -CN, nitro, C₁-C₄alkyl-, C₂-C₄alkenyl-, C₂-C₄alkynyl-, C₁-C₄haloalkyl-, C₁-C₄ alkoxy-, C₁-C₄haloalkoxy-, -S(O)ₙC₁-C₄alkyl and C₃-C₆cycloalkyl-;
R² is selected from the group consisting of halogen, -CN, nitro, C₁-C₄alkyl, C₂-C₄alkenyl-, C₂-C₄alkynyl-, C₁-C₄haloalkyl-, C₁-C₄alkoxy-, C₁-C₄haloalkoxy-, - S(O)ₙC₁-C₄alkyl and C₃-C₆cycloalkyl-;
m is 1, 2, 3 or 4;
n is 0, 1 or 2;
p is 0, 1 or 2;
R³ is selected from C₃-C₆cycloalkyl, phenyl and a 5 or 6 membered heteroaryl which comprises from 1 to 4 heteroatoms each independently selected from the group consisting of oxygen, nitrogen and sulphur, and wherein said phenyl or heteroaryl groups are optionally substituted by one, two or three substituents independently selected from the group consisting of halogen, cyano, C₁-C₄ alkyl, C₁-C₄ haloalkyl, C₁-C₄ alkoxy and C₁-C₄ haloalkoxy;
or an agriculturally acceptable salt thereof.

2. A compound according to claim 1, wherein the compound of Formula (I) is a compound of Formula (la):

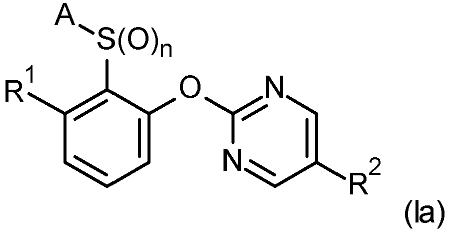

3. A compound according to any one of the previous claims, wherein A is selected from the group consisting of C₃-C₈ alkyl, C₃-C₈ alkenyl, C₃-C₈ alkynyl, C₃-C₈ haloalkyl, C₃-C₈ haloalkenyl, C₃-C₈ haloalkynyl, C₁-C₄alkoxy-C₁-C₃alkyl-, C₁-C₄haloalkoxy-C₁-C₃alkyl- and C₁-C₄alkoxy-C₁-C₃haloalkyl-.

4. A compound according to any one of the previous claims, wherein A is selected from the group consisting of C₃-C₈ alkyl, C₃-C₈ haloalkyl, C₃-C₈ haloalkenyl, C₃-C₈ haloalkynyl and C₁-C₄haloalkoxy-C₁-C₃alkyl-;

5. A compound according to any one of the previous claims, wherein R¹ is halogen or -CN.

6. A compound according to any one of the previous claims, wherein R² is selected from the group consisting of halogen, C₁-C₄ alkyl, C₂-C₄ alkenyl, C₂-C₄ alkynyl, C₁-C₄ haloalkyl, C₁-C₄ alkoxy and C₁-C₄ haloalkoxy.

7. A compound according to any one of the previous claims, wherein R² is selected from the group consisting of methyl, methoxy- and halogen.

8. A compound according to any one of the previous claims, wherein R² is halogen.

9. A compound according to any one of the previous claims, wherein R² is chloro.

10. A herbicidal composition comprising a compound according to any one of the previous claims and an agriculturally acceptable formulation adjuvant.

11. A herbicidal composition according to claim 10, further comprising at least one additional pesticide.

12. A herbicidal composition according to claim 11, wherein the additional pesticide is a herbicide or herbicide safener.

13. A method of controlling weeds at a locus comprising application to the locus of a weed controlling amount of a composition according to any one of claims 10 to 12.

14. Use of a compound of Formula (I) as defined in claim 1 as a herbicide.

## Patentansprüche

1. Verbindung der Formel (I): 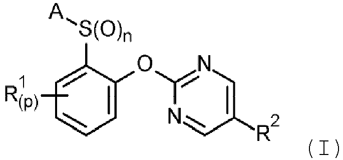 wobei
A aus der Gruppe bestehend aus C₃-C₈-Alkyl, C₃-C₈-Alkenyl, C₃-C₈-Alkinyl, C₃-C₈-Halogenalkyl, C₃-C₈-Halogenalkenyl, C₃-C₈ Halogenalkinyl, C₁-C₄-Alkoxy-C₁-C₃-alkyl-, C₁-C₄-Halogenalkoxy-C₁-C₃-alkyl-, C₁-C₄-Alkoxy-C₁-C₃-halogenalkyl- und -(CH₂)ₘR³ ausgewählt ist;
R¹ unabhängig aus der Gruppe bestehend aus Halogen, -CN, Nitro, C₁-C₄-Alkyl, C₂-C₄-Alkenyl, C₂-C₄-Alkinyl, C₁-C₄-Halogenalkyl, C₁-C₄-Alkoxy-, C₁-C₄-Halogenalkoxy-, - S(O)ₙC₁-C₄-Alkyl und C₃-C₆-Cycloalkyl- ausgewählt ist;
R² aus der Gruppe bestehend aus Halogen, -CN, Nitro, C₁-C₄-Alkyl, C₂-C₄-Alkenyl, C₂-C₄-Alkinyl, C₁-C₄-Halogenalkyl, C₁-C₄-Alkoxy-, C₁-C₄-Halogenalkoxy-, - S(O)ₙC₁-C₄-Alkyl und C₃-C₆-Cycloalkyl- ausgewählt ist;
m für 1, 2, 3 oder 4 steht;
n für 0, 1 oder 2 steht;
p für 0, 1 oder 2 steht;
R³ aus C₃-C₆-Cycloalkyl, Phenyl und einem 5- oder 6-gliedrigen Heteroaryl mit 1 bis 4 Heteroatomen, die jeweils unabhängig aus der Gruppe bestehend aus Sauerstoff, Stickstoff und Schwefel ausgewählt sind, ausgewählt ist, und wobei die Phenyl- oder Heteroarylgruppen gegebenenfalls durch einen, zwei oder drei Substituenten, die unabhängig aus der Gruppe bestehend aus Halogen, Cyano, C₁-C₄-Alkyl, C₁-C₄-Halogenalkyl, C₁-C₄-Alkoxy und C₁-C₄-Halogenalkyl ausgewählt sind, substituiert sind;
oder ein landwirtschaftlich unbedenkliches Salz davon.

2. Verbindung nach Anspruch 1, wobei es sich bei der Verbindung der Formel (I) um eine Verbindung der Formel (Ia) handelt:

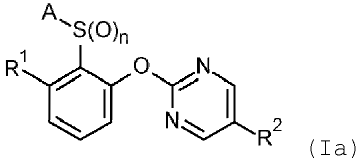

3. Verbindung nach einem der vorhergehenden Ansprüche, wobei A aus der Gruppe bestehend aus C₃-C₈-Alkyl, C₃-C₈-Alkenyl, C₃-C₈-Alkinyl, C₃-C₈-Halogenalkyl, C₃-C₈-Halogenalkenyl, C₃-C₈-Halogenalkinyl, C₁-C₄-Alkoxy-C₁-C₃-alkyl-, C₁-C₄-Halogenalkoxy-C₁-C₃-alkyl- und C₁-C₄-Alkoxy-C₁-C₃-halogenalkyl- ausgewählt ist.

4. Verbindung nach einem der vorhergehenden Ansprüche, wobei A aus der Gruppe bestehend aus C₃-C₈-Alkyl, C₃-C₈-Halogenalkyl, C₃-C₈-Halogenalkenyl, C₃-C₈-Halogenalkinyl und C₁-C₄-Halogenalkoxy-C₁-C₃-alkyl- ausgewählt ist.

5. Verbindung nach einem der vorhergehenden Ansprüche, wobei R¹ für Halogen oder -CN steht.

6. Verbindung nach einem der vorhergehenden Ansprüche, wobei R² aus der Gruppe bestehend aus Halogen, C₁-C₄-Alkyl, C₂-C₄-Alkenyl, C₂-C₄-Alkinyl, C₁-C₄-Halogenalkyl, C₁-C₄-Alkoxy und C₁-C₄-Halogenalkoxy ausgewählt ist.

7. Verbindung nach einem der vorhergehenden Ansprüche, wobei R² aus der Gruppe bestehend aus Methyl, Methoxy- und Halogen ausgewählt ist.

8. Verbindung nach einem der vorhergehenden Ansprüche, wobei R² für Halogen steht.

9. Verbindung nach einem der vorhergehenden Ansprüche, wobei R² für Chlor steht.

10. Herbizide Zusammensetzung, umfassend eine Verbindung nach einem der vorhergehenden Ansprüche und ein landwirtschaftlich unbedenkliches Formulierungshilfsmittel.

11. Herbizide Zusammensetzung nach Anspruch 10, ferner umfassend mindestens ein zusätzliches Pestizid.

12. Herbizide Zusammensetzung nach Anspruch 11, wobei es sich bei dem zusätzlichen Pestizid um ein Herbizid oder einen Herbizid-Safener handelt.

13. Verfahren zur Bekämpfung von Unkräutern an einem Standort, bei dem man eine unkrautbekämpfende Menge einer Zusammensetzung nach einem der Ansprüche 10 bis 12 auf den Standort ausbringt.

14. Verwendung einer Verbindung der Formel (I) gemäß Anspruch 1 als Herbizid.

## Revendications

1. Composé de formule (I) : 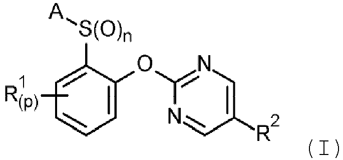
A étant choisi dans le groupe constitué de C₃-C₈ alkyle, C₃-C₈ alcényle, C₃-C₈ alcynyle, C₃-C₈ halogénoalkyle, C₃-C₈ halogénoalcényle, C₃-C₈ halogénoalcynyle, C₁-C₄alcoxy-C₁-C₃alkyle-, C₁-C₄halogénoalcoxy-C₁-C₃alkyle-, C₁-C₄alcoxy-C₁-C₃halogénoalkyl- et -(CH₂)R³ ;
R¹ étant indépendamment choisi dans le groupe constitué d'halogène, -CN, nitro, C₁-C₄alkyle-, C₂-C₄alcényle-, C₂-C₄alcynyle-, C₁-C₄halogénoalkyle-, C₁-C₄ alcoxy-, C₁-C₄halogénoalcoxy-, -S(O)ₙC₁-C₄alkyle et C₃-C₆cycloalkyle- ;
R² étant choisi dans le groupe constitué d'halogène, -CN, nitro, C₁-C₄alkyle, C₂-C₄alcényle-, C₂-C₄alcynyle-, C₁-C₄halogénoalkyle-, C₁-C₄alcoxy-, C₁-C₄halogénoalcoxy-, - S(O)ₙC₁-C₄alkyle et C₃-C₆cycloalkyle- ;
m étant 1, 2, 3 ou 4 ;
n étant 0, 1 ou 2 ;
p étant 0, 1 ou 2 ;
R³ étant choisi parmi C₃-C₆cycloalkyle, phényle et un hétéroaryle à 5 ou 6 chaînons qui comprend de 1 à 4 hétéroatomes chacun indépendamment choisi dans le groupe constitué d'oxygène, azote et soufre, et lesdits groupes phényle ou hétéroaryle étant éventuellement substitués par un, deux ou trois substituants indépendamment choisis dans le groupe constitué d'halogène, cyano, C₁-C₄ alkyle, C₁-C₄ halogénoalkyle, C₁-C₄ alcoxy et C₁-C₄ halogénoalcoxy ;
ou sel acceptable sur le plan agricole correspondant.

2. Composé selon la revendication 1, le composé de formule (I) étant un composé de formule (Ia) :

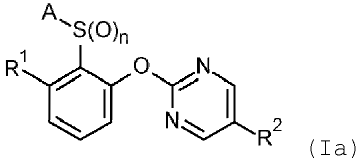

3. Composé selon l'une quelconque des revendications précédentes, A étant choisi dans le groupe constitué de C₃-C₈ alkyle, C₃-C₈ alcényle, C₃-C₈ alcynyle, C₃-C₈ halogénoalkyle, C₃-C₈ halogénoalcényle, C₃-C₈ halogénoalcynyle, C₁-C₄alcoxy-C₁-C₃alkyle-, C₁-C₄halogénoalcoxy-C₁-C₃alkyl- et C₁-C₄alcoxy-C₁-C₃halogénoalkyle-.

4. Composé selon l'une quelconque des revendications précédentes, A étant choisi dans le groupe constitué de C₃-C₈ alkyle, C₃-C₈ halogénoalkyle, C₃-C₈ halogénoalcényle, C₃-C₈ halogénoalcynyle et C₁-C₄halogénoalcoxy-C₁-C₃alkyle-.

5. Composé selon l'une quelconque des revendications précédentes, R¹ étant halogène ou -CN.

6. Composé selon l'une quelconque des revendications précédentes, R² étant choisi dans le groupe constitué d'halogène, C₁-C₄ alkyle, C₂-C₄ alcényle, C₂-C₄ alcynyle, C₁-C₄ halogénoalkyle, C₁-C₄ alcoxy et C₁-C₄ halogénoalcoxy.

7. Composé selon l'une quelconque des revendications précédentes, R² étant choisi dans le groupe constitué de méthyle, méthoxy- et halogène.

8. Composé selon l'une quelconque des revendications précédentes, R² étant halogène.

9. Composé selon l'une quelconque des revendications précédentes, R² étant chloro.

10. Composition herbicide comprenant un composé selon l'une quelconque des revendications précédentes et un adjuvant de formulation acceptable sur le plan agricole.

11. Composition herbicide selon la revendication 10, comprenant en outre au moins un pesticide supplémentaire.

12. Composition herbicide selon la revendication 11, le pesticide supplémentaire étant un herbicide ou un phytoprotecteur herbicide.

13. Procédé de lutte contre des adventices au niveau d'un lieu comprenant une application sur le lieu d'une quantité de lutte contre des adventices d'une composition selon l'une quelconque des revendications 10 à 12.

14. Utilisation d'un composé de formule (I) tel que défini dans la revendication 1 comme un herbicide.
